# EUROPEAN PATENT SPECIFICATION

(11) **EP 3 599 877 B1**
(45) Date of publication and mention of the grant of the patent: **06.07.2022**
(21) Application number: 18711573.8
(22) Date of filing: 21.03.2018
(51) Int. Cl.: A23C 9/123, C12N 15/74, A23C 13/16, A23C 17/02, A23C 19/032, A23C 21/02

(54) **LACTIC ACID BACTERIA COMPOSITION FOR PREPARING FERMENTED FOOD PRODUCTS WITH INCREASED NATURAL SWEETNESS AND FLAVOR**
MILCHSÄUREBAKTERIENZUSAMMENSETZUNG ZUR HERSTELLUNG FERMENTIERTER LEBENSMITTELPRODUKTE MIT ERHÖHTER NATÜRLICHER SÜSSE UND ERHÖHTEM NATÜRLICHEM GESCHMACK
COMPOSITION DE BACTÉRIES D'ACIDE LACTIQUE POUR PRÉPARER DES PRODUITS ALIMENTAIRES FERMENTÉS À SUCROSITÉ NATURELLE ET SAVEUR ACCRUES

(30) Priority: 28.03.2017 EP 17163311
(43) Date of publication of application: 05.02.2020
(73) Proprietor: Chr. Hansen A/S, 2970 Hoersholm (DK)
(72) Inventor: BLOCH, Sonja, 1307 Copenhagen K (DK); BUCHHORN, Gaelle Lettier, 2880 Virum (DK)
(86) International application number: PCT/EP2018/057153
(87) International publication number: WO 2018/177835

(56) References cited:
- WO-A1-2015/193449
- WO-A1-2015/193459
- US-A1- 2005 196 388
- US-A1- 2015 086 675
- KIM I. SØRENSEN ET AL: "ABSTRACT", APPLIED AND ENVIRONMENTAL MICROBIOLOGY, vol. 82, no. 12, 15 June 2016 (2016-06-15) , pages 3683-3692, XP055337824, ISSN: 0099-2240, DOI: 10.1128/AEM.00462-16
- ROBERT W HUTKINS ET AL: "Use of Galactose-Fermenting Streptococcus thermophilus in the Manufacture of Swiss, Mozzarella, and Short-Method Cheddar Cheese", JOURNAL OF DAIRY SCIENCE., vol. 69, no. 1, 1986, pages 1-8, XP055385723, US ISSN: 0022-0302

### SEQUENCE LISTING

<110> Chr. Hansen A/S
<120> Lactic acid bacteria for preparing fermented food products with increased natural sweetness and high texture
<130> P6233PC00
<160> 6
<170> PatentIn version 3.5
<210> 1
   <211> 966
   <212> DNA
   <213> Streptococcus thermophilus
<400> 1
   atgagtaaga aactcttagg tattgacctt ggtggaacaa ctgttaagtt tggtattttg        60
   actgcagatg gtgaagttca agaaaaatgg gctattgaaa caaatacgtt tgaaaatggt       120
   agccacattg ttcctgacat tgtagaatct ttgaaacacc gtttggaatt gtatggactt       180
   actgctgaag attttattgg aattggtatg ggatctccag gtgcagttga ccgagaaaat       240
   aaaacagtaa cgggtgcctt taacttgaac tgggcagaaa ctcaagaagt tggctctgtt       300
   attgaaaaag aacttggtat tccattcgct attgataatg atgctaatgt ggctgcactg       360
   ggtgaacgtt gggttggtgc tggtgctaac aatcggaatg ttgtctttat aacattgggt       420
   acaggtgttg gtggcggtgt tatcgctgat ggtaacttaa ttcatggtgt tgccggtgct       480
   ggtggggaaa ttggtcacat tattgttgaa cctgacacag gatttgagtg tacttgcgga       540
   aacaaggggt gtctggaaac tgtagcttca gcaacaggta ttgtacgtgt agcacatcat       600
   ttggcagaaa aatacgaagg aaactcttct attaaagctg ctgtagacaa tggtgagttt       660
   gtgacaagta aagatattat cgtagctgct actgaaggtg ataagtttgc tgacagcatt       720
   gttgataaag tctctaaata cctcagactt gcaacagcaa acatctcaaa cattcttaac       780
   ccagattctg tcgttatcgg tggtggtgtt tctgccgcag gagaattctt gcgtagtcgt       840
   gttgaaggat actttacacg ttatgcattc ccacaagttc gccgtacaac aaaagtgaaa       900
   ttagcggagc ttggaaatga tgcaggaatc attggagctg ctagtcttgc ttatagtatt       960
   gacaaa                                                                  966
<210> 2
   <211> 322
   <212> PRT
   <213> Streptococcus thermophilus
<400> 2
   Met Ser Lys Lys Leu Leu Gly Ile Asp Leu Gly Gly Thr Thr Val Lys
   1               5                   10                  15
   Phe Gly Ile Leu Thr Ala Asp Gly Glu Val Gln Glu Lys Trp Ala Ile
               20                  25                  30
   Glu Thr Asn Thr Phe Glu Asn Gly Ser His Ile Val Pro Asp Ile Val
           35                  40                  45
   Glu Ser Leu Lys His Arg Leu Glu Leu Tyr Gly Leu Thr Ala Glu Asp
       50                  55                  60
   Phe Ile Gly Ile Gly Met Gly Ser Pro Gly Ala Val Asp Arg Glu Asn
   65                  70                  75                  80
   Lys Thr Val Thr Gly Ala Phe Asn Leu Asn Trp Ala Glu Thr Gln Glu
                   85                  90                  95
   Val Gly Ser Val Ile Glu Lys Glu Leu Gly Ile Pro Phe Ala Ile Asp
               100                 105                 110
   Asn Asp Ala Asn Val Ala Ala Leu Gly Glu Arg Trp Val Gly Ala Gly
           115                 120                 125
   Ala Asn Asn Arg Asn Val Val Phe Ile Thr Leu Gly Thr Gly Val Gly
       130                 135                 140
   Gly Gly Val Ile Ala Asp Gly Asn Leu Ile His Gly Val Ala Gly Ala
   145                 150                 155                 160
   Gly Gly Glu Ile Gly His Ile Ile Val Glu Pro Asp Thr Gly Phe Glu
                   165                 170                 175
   Cys Thr Cys Gly Asn Lys Gly Cys Leu Glu Thr Val Ala Ser Ala Thr
               180                 185                 190
   Gly Ile Val Arg Val Ala His His Leu Ala Glu Lys Tyr Glu Gly Asn
           195                 200                 205
   Ser Ser Ile Lys Ala Ala Val Asp Asn Gly Glu Phe Val Thr Ser Lys
       210                 215                 220
   Asp Ile Ile Val Ala Ala Thr Glu Gly Asp Lys Phe Ala Asp Ser Ile
   225                 230                 235                 240
   Val Asp Lys Val Ser Lys Tyr Leu Arg Leu Ala Thr Ala Asn Ile Ser
                   245                 250                 255
   Asn Ile Leu Asn Pro Asp Ser Val Val Ile Gly Gly Gly Val Ser Ala
   260 265 270
   Ala Gly Glu Phe Leu Arg Ser Arg Val Glu Gly Tyr Phe Thr Arg Tyr
   275 280 285
   Ala Phe Pro Gln Val Arg Arg Thr Thr Lys Val Lys Leu Ala Glu Leu
   290 295 300
   Gly Asn Asp Ala Gly Ile Ile Gly Ala Ala Ser Leu Ala Tyr Ser Ile
   305 310 315 320
   Asp Lys
<210> 3
   <211> 909
   <212> DNA
   <213> Streptococcus thermophilus
<400> 3
   atggctgaaa aaattcaatt atctcaagcg gatcgtaaaa aggtttggtg gcgctcacaa      60
   ttcttgcaag gtgcatggaa ctatgaacgt atgcaaaact tgggttgggc ttactcactc     120
   attcctgcta tcaaaaaact ttatactaac aaagaggacc aagccgcagc tcttaaacgt     180
   cacttggaat tcttcaacac tcacccttac gtagctgctc ctatcatagg ggttccctta     240
   gctcttgaag aagaaaaagc taatggtact gaaatcgaag atgcggctat ccaaggggtt     300
   aaaatcggta tgatgggtcc acttgccggt atcggtgacc ctgtcttctg gttcacaatt     360
   cgtccaattc ttggtgccct tggtgcatca ttggcacaag ctggtaacat tgctggtcca     420
   cttatcttct tcattggttg gaaccttatc cgcatggcct tcttgtggta cactcaagaa     480
   cttggttaca aagcaggttc agaaatcact aaagacatat ctggtggtat cttgaaagat     540
   attactaaag gggcatcaat acttggtatg ttcatcttgg ccgtcctcgt tgaacgttgg     600
   gtatctgtcg tcttcactgt aaagcttcca ggtaaagttt tgcctaaagg tgcttatatt     660
   gaatggccaa aaggatatgt tactggtgac caactaaaaa ctatccttgg tcaagtcaac     720
   gataagctta gctttgataa gattcaagtc gataccctac aaaaacaatt ggattcatta     780
   attccaggtt tgacgggact tctccttact tttgcatgta tgtggttgct taagaagaaa     840
   gtttcaccaa tcacaatcat catcggactc tttgtagttg gtattattgc aagcttcttc     900
   ggaatcatg                                                             909
<210> 4
   <211> 303
   <212> PRT
   <213> Streptococcus thermophilus
<400> 4
   Met Ala Glu Lys Ile Gln Leu Ser Gln Ala Asp Arg Lys Lys Val Trp
   1               5                   10                  15
   Trp Arg Ser Gln Phe Leu Gln Gly Ala Trp Asn Tyr Glu Arg Met Gln
               20                  25                  30
   Asn Leu Gly Trp Ala Tyr Ser Leu Ile Pro Ala Ile Lys Lys Leu Tyr
           35                  40                  45
   Thr Asn Lys Glu Asp Gln Ala Ala Ala Leu Lys Arg His Leu Glu Phe
       50                  55                  60
   Phe Asn Thr His Pro Tyr Val Ala Ala Pro Ile Ile Gly Val Pro Leu
   65                  70                  75                  80
   Ala Leu Glu Glu Glu Lys Ala Asn Gly Thr Glu Ile Glu Asp Ala Ala
                   85                  90                  95
   Ile Gln Gly Val Lys Ile Gly Met Met Gly Pro Leu Ala Gly Ile Gly
               100                 105                 110
   Asp Pro Val Phe Trp Phe Thr Ile Arg Pro Ile Leu Gly Ala Leu Gly
           115                 120                 125
   Ala Ser Leu Ala Gln Ala Gly Asn Ile Ala Gly Pro Leu Ile Phe Phe
       130                 135                 140
   Ile Gly Trp Asn Leu Ile Arg Met Ala Phe Leu Trp Tyr Thr Gln Glu
   145                 150                 155                 160
   Leu Gly Tyr Lys Ala Gly Ser Glu Ile Thr Lys Asp Ile Ser Gly Gly
                   165                 170                 175
   Ile Leu Lys Asp Ile Thr Lys Gly Ala Ser Ile Leu Gly Met Phe Ile
               180                 185                 190
   Leu Ala Val Leu Val Glu Arg Trp Val Ser Val Val Phe Thr Val Lys
           195                 200                 205
   Leu Pro Gly Lys Val Leu Pro Lys Gly Ala Tyr Ile Glu Trp Pro Lys
       210                 215                 220
   Gly Tyr Val Thr Gly Asp Gln Leu Lys Thr Ile Leu Gly Gln Val Asn
   225                 230                 235                 240
   Asp Lys Leu Ser Phe Asp Lys Ile Gln Val Asp Thr Leu Gln Lys Gln
                   245                 250                 255
   Leu Asp Ser Leu Ile Pro Gly Leu Thr Gly Leu Leu Leu Thr Phe Ala
               260                 265                 270
   Cys Met Trp Leu Leu Lys Lys Lys Val Ser Pro Ile Thr Ile Ile Ile
           275                 280                 285
   Gly Leu Phe Val Val Gly Ile Ile Ala Ser Phe Phe Gly Ile Met
       290                 295                 300
<210> 5
   <211> 828
   <212> DNA
   <213> Streptococcus thermophilus
<400> 5
   atgtcagata tgtcaattat ttctgcgatt ttggtcgtag ctgttgcctt ccttgctggt      60
   cttgaaagta tccttgacca attccaattc caccaaccac ttgttgcatg taccctcatc     120
   ggtgctgcca caggtaacct cactgcaggt atcatgcttg gtggttctct tcaaatgatt     180
   acccttgctt gggcaaacat cggtgctgcc gtagctcctg acgttgccct tgcatctgtt     240
   gccgctgcca tcattttggt taaaggtggt aaatttacag ctgaaggtat cggtgttgcg     300
   attgcaatag ctatcctgct tgcagttgca ggtctcttcc taactatgcc tgttcgtaca     360
   gcatctattg cctttgttca tgctgcagat aaagctgcag aacacggaaa catcgctggt     420
   gttgaacgtg catactacct cgctctcctt cttcaaggtt tgcgtattgc tgtgccagca     480
   gcccttcttc ttgccatccc ggcccaatct gttcaacatg cccttggctt gatgcctgac     540
   tggctcaccc atggtttggt tgtcggtggt ggtatggtcg tagccgttgg ttacgccatg     600
   attatcaata tgatggctac tcgtgaagtt tggccattct tcgccattgg ttttgctttg     660
   gcagcaatta gccaattgac acttatcgct cttagtacca ttggtgttgc catcgccttc     720
   atctacctca acctttctaa acaaggtggc ggaaatggtg gcggaaatgg tggcggaact     780
   tcatctggtt caggcgaccc aatcggcgat atcttggaag actactag                  828
<210> 6
   <211> 275
   <212> **PRT**
   <213> Streptococcus thermophilus
<400> 6
   Met Ser Asp Met Ser Ile Ile Ser Ala Ile Leu Val Val Ala Val Ala
   1               5                   10                  15
   Phe Leu Ala Gly Leu Glu Ser Ile Leu Asp Gln Phe Gln Phe His Gln
               20                  25                  30
   Pro Leu Val Ala Cys Thr Leu Ile Gly Ala Ala Thr Gly Asn Leu Thr
           35                  40                  45
   Ala Gly Ile Met Leu Gly Gly Ser Leu Gln Met Ile Thr Leu Ala Trp
       50                  55                  60
   Ala Asn Ile Gly Ala Ala Val Ala Pro Asp Val Ala Leu Ala Ser Val
   65                  70                  75                  80
   Ala Ala Ala Ile Ile Leu Val Lys Gly Gly Lys Phe Thr Ala Glu Gly
                   85                  90                  95
   Ile Gly Val Ala Ile Ala Ile Ala Ile Leu Leu Ala Val Ala Gly Leu
               100                 105                 110
   Phe Leu Thr Met Pro Val Arg Thr Ala Ser Ile Ala Phe Val His Ala
           115                 120                 125
   Ala Asp Lys Ala Ala Glu His Gly Asn Ile Ala Gly Val Glu Arg Ala
       130                 135                 140
   Tyr Tyr Leu Ala Leu Leu Leu Gln Gly Leu Arg Ile Ala Val Pro Ala
   145                 150                 155                 160
   Ala Leu Leu Leu Ala Ile Pro Ala Gln Ser Val Gln His Ala Leu Gly
                   165                 170                 175
   Leu Met Pro Asp Trp Leu Thr His Gly Leu Val Val Gly Gly Gly Met
               180                 185                 190
   Val Val Ala Val Gly Tyr Ala Met Ile Ile Asn Met Met Ala Thr Arg
           195                 200                 205
   Glu Val Trp Pro Phe Phe Ala Ile Gly Phe Ala Leu Ala Ala Ile Ser
       210                 215                 220
   Gln Leu Thr Leu Ile Ala Leu Ser Thr Ile Gly Val Ala Ile Ala Phe
   225                 230                 235                 240
   Ile Tyr Leu Asn Leu Ser Lys Gln Gly Gly Gly Asn Gly Gly Gly Asn
                   245                 250                 255
   Gly Gly Gly Thr Ser Ser Gly Ser Gly Asp Pro Ile Gly Asp Ile Leu
               260                 265                 270
   Glu Asp Tyr

## Description

The present invention relates to a composition according to claim 1 for producing a fermented milk product comprising at least one *Streptococcus thermophilus* (St) strain, wherein the St strain is galactose-fermenting, wherein the strain carries a mutation in the DNA sequence of the *glcK* gene encoding a glucokinase protein, wherein the mutation inactivates the glucokinase protein or has a negative effect on expression of the gene, and (ii) at least one *Lactobacillus delbrueckii* subsp. *bulgaricus* (Lb) strain, wherein the Lb strain is lactose-deficient and capable of metabolizing glucose. Such a composition is used for producing fermented milk products with an increased sweetness.

### BACKGROUND OF THE INVENTION

Pure fermented milk products are recognized by a tart or sour taste as a result of the conversion of lactose to lactic acid by lactic acid bacteria during fermentation. They are, therefore, often sweetened by the addition of fruit, honey, sugar or artificial sweeteners to accommodate the customers' desire for a sweeter tasting product.

The food industry has an increasingly high demand for low-calorie sweet-tasting food products in order to help overcome the overweight and obesity problems that have become so prevalent in the last 20 years. Sweetness, usually regarded as a pleasurable sensation, is produced by the presence of sugars and a few other substances. The perception of sugars is very different. Using sucrose as a 100 reference, the sweetness of lactose is 16, of galactose 32 and of glucose 74 (Godshall (1988). Food Technology 42(11):71-78). Glucose is thus perceived more than 4 times sweeter than lactose while still having approximately the same level of calories.

Sugar in fermented food products is more often being replaced with sweeteners such as aspartame, acesulfame K, sucralose and saccharin which can provide the sweetness with a lower intake of calories. However, the use of artificial sweeteners may result in an off-taste and several studies indicating that the consumption of artificial sweeteners are connected with drawbacks, such as increasing hunger, allergies, cancer etc., have contributed to consumer's preference for fermented milk products which only contain natural sweeteners or, preferably, contain no added sweetener.

Thus, a special challenge lies in developing fermented milk products where the natural (inner) sweetness is high.

The acidity of fermented milk products depend in large part on the lactic acid bacteria present and the process parameters used for preparing the fermented milk product.

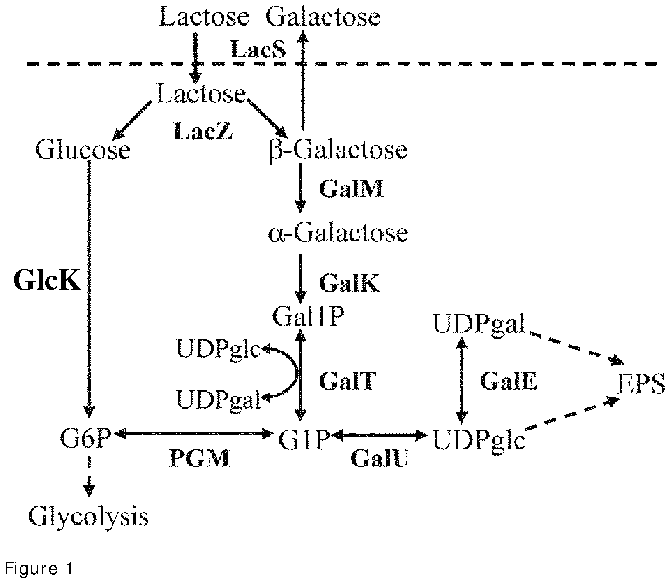

Fermentation of the disaccharide lactose is very much studied in lactic acid bacteria because it is the major carbon source in milk. In many species, lactose is cleaved by β-galactosidase into glucose and galactose after uptake. The glucose is phosphorylated by glucokinase to glucose-6-phosphate and fermented via the Embden-Meyerhof-Parnas pathway (glycolysis) by most lactic acid bacteria (Figure 1).

*Streptococcus thermophilus* is one of the most widely used lactic acid bacteria for commercial thermophilic milk fermentation where the organism is normally used as part of a mixed starter culture, the other component being a *Lactobacillus* sp., e.g. *Lactobacillus delbrueckii* subsp. *bulgaricus* for yoghurt or *Lactobacillus helveticus* for Swiss-type cheese.

The legal definition of yoghurt in many countries requires *Streptococcus thermophilus* alongside *Lactobacillus delbrueckii* subsp. *bulgaricus.* Both species generate desirable amounts of acetaldehyde, an important flavor component in yoghurt.

Lactose and sucrose are fermented more readily by *Streptococcus thermophilus* than their component monosaccharides. In the presence of excess galactose only the glucose portion of the lactose molecule is fermented and galactose accumulates in fermented milk products when *Streptococcus thermophilus* is used. In yoghurt wherein high acid concentrations limit the fermentation, free galactose remains while the free galactose produced in the early stages of Swiss cheese manufacture is later fermented by *Lactobacillus helveticus.*

However, galactose fermenting strains of both *Streptococcus thermophilus* and *Lactobacillus delbrueckii* subsp. *bulgaricus* have been reported by several researchers (Hutkins et al. (1986) J. Dairy Sci. 69(1): 1-8; Vaillancourt et al. (2002) J. Bacteriol. 184(3); 785-793) and in WO 2011/026863 (Chr. Hansen) is described a method for obtaining *Streptococcus thermophilus* strains which are galactose fermenting.

In order to meet the requirements of the food industry, it has become relevant to propose new strains, in particular *Streptococcus thermophilus* strains and *Lactobacillus delbrueckii* subsp. *bulgaricus* strains, which provide more natural sweetness without extra calories directly into the fermented product (inner sweetness) by excretion of glucose.

Pool et al. (2006. Metabolic Engineering 8(5); 456-464) discloses *Lactococcus lactis* strain in which the glucose metabolism is completely disrupted by deletion of the genes coding for glucokinase, EII(man/glc) and the newly discovered glucose-PTS EII(cel). The construction method is genetic recombination for generation of all the mutations and the resulting strain is consequently a genetically modified organism (GMO) that at present cannot be used in food products.

Thompson et al. (1985. J Bacteriol. 162(1); 217-223) studied the lactose metabolism in *Streptococcus lactis* (today renamed *Lactococcus lactis).* In this work 2-deoxyglucose was used to obtain a mutant in the mannose-PTS system. Subsequently, this mutant was mutagenized using UV mutagenesis followed by screening for glucose-negative colonies by replica plating. In this way a double mutant (mannose PTS and glucokinase) was isolated. This double mutant was used to study the mechanisms involved in the regulation of lactose fermentation by "starter" organisms. These mutants have several disadvantages compared to their parent strain which makes them unsuitable for inclusion in a commercial starter culture. The cell yield of the mutants was half that of the parent strain per mole of lactose fermented and the doubling time was significantly increased in the mutants when grown on lactose. Likewise, the yield of lactic acid was half that of the parent strain per mole of lactose fermented. The behavior of these strains in milk was not analyzed but it is anticipated that the rate of acidification would be significantly reduced.

In addition, *Lactococcus lactis* is generally not chosen for acetaldehyde production and does not contribute to the fulfillment of the requirements for the legal definition of yoghurt.

Chervaux et al. (2000. Appl. And Environ. Microbiol., 66, 5306-5311), studied the physiology of *Lactobacillus delbrueckii* subsp. *bulgaricus* strains in a novel chemically defined medium and isolated 2-deoxyglucose-resistant mutants that were deficient in glucose fermentation. Several different phenotypes were observed and strain-specific effects were reported.

WO2013/160413 discloses *Streptococcus thermophilus* strains and *Lactobacillus delbrueckii* subsp. *bulgaricus* strains with enhanced properties for natural sweetening of food products and for decreasing the lactose content of fermented milk, and method of screening and isolating such strains.

WO2015/193449 discloses a method of producing a fermented milk product with a very low concentration of lactose using a combination of lactase and lactic acid bacteria with a deficiency in glucose metabolism.

WO2015/193459 discloses a method of producing a fermented milk product with a reduced level of post-acidification, wherein one embodiment uses a mixture of four lactic acid bacteria strains with a deficiency in glucose metabolism, and wherein another embodiment uses a mixture of lactose-deficient lactic acid bacteria strains.

The object of the present invention is to provide a composition for producing a fermented milk product comprising at least one galactose-fermenting, glucose-deficient *Streptococcus thermophilus* strain and at least one lactose-deficient, glucose-fermenting *Lactobacillus delbrueckii* subsp. *bulgaricus* strain, wherein the composition produces a fermented milk product with an improved flavor.

### SUMMARY OF I NVENTI ON

This object is achieved with the present invention, which is directed to a composition for producing a fermented milk product comprising (i) at least one *Streptococcus thermophilus* (St) strain, wherein the St strain is galactose-fermenting, wherein the strain carries a mutation in the DNA sequence of the *glcK* gene encoding a glucokinase protein, wherein the mutation inactivates the glucokinase protein or has a negative effect on expression of the gene, and (ii) at least one *Lactobacillus delbrueckii* subsp. *bulgaricus* (Lb) strain, wherein the Lb strain is lactose-deficient and capable of metabolizing glucose.

It is well known that for conventional starter cultures for producing fermented milk products comprising a *Streptococcus thermophilus* strain and a *Lactobacillus delbrueckii* subsp. *bulgaricus* strain, there is a risk of formation of undesired off-flavors, such as free amino acids, e.g. glutamic acid. It is further well known that the off-flavors are formed by the *Lactobacillus delbrueckii* subsp. *bulgaricus* strain, and that the level of off-flavors formed depends on the level of growth of the said strain, which varies depending on a number of factors, such as the inoculation level of the *Lactobacillus delbrueckii* subsp. *bulgaricus* strain, the type of growth media and growth conditions of the fermentation as well as the specific *Streptococcus thermophilus* strain and *Lactobacillus delbrueckii* subsp. *bulgaricus* strain used in the starter culture. It has now been found that for starter cultures for producing a fermented milk product with enhanced sweetness comprising at least one glucose-deficient *Streptococcus thermophilus* strain and at least one *Lactobacillus delbrueckii* subsp. *bulgaricus* strain, the formation of undesired off-flavors is at a particular high level. This is consistent with the fact that in such starter cultures the *Lactobacillus delbrueckii* subsp. *bulgaricus* strain grows to a particular high cell count.

It has surprisingly been found that the problem of off-flavors using starter cultures with glucose-deficient *Streptococcus thermophilus* strain is solved by using lactose-deficient, glucose-positive *Lactobacillus delbrueckii* subsp. *bulgaricus* strain. Also, it has been found that said lactose-deficient *Lactobacillus delbrueckii* subsp. *bulgaricus* strain used in the composition of the invention does not result in any post-acidification. Finally, it has been found that said lactose-deficient *Lactobacillus delbrueckii* subsp. *bulgaricus* in combination with glucose-deficient *Streptococcus thermophilus* strains produces a very high cell count as compared to conventional, i.e. lactose-positive, glucose-positive, *Lactobacillus delbrueckii* subsp. *bulgaricus* strains.

It is highly surprising that it is possible to avoid the formation of off-flavors from a lactose-deficient, glucose-positive *Lactobacillus delbrueckii* subsp. *bulgaricus* strain and to avoid post-acidification in view of the fact that the *Lactobacillus delbrueckii* subsp. *bulgaricus* strain is glucose-positive and present in a fermented milk product containing glucose, where in theory the strain would be able to continue growth during storage of the fermented milk product.

Moreover, it is even more surprising that it is possible to avoid the formation of off-flavors from the *Lactobacillus delbrueckii* subsp. *bulgaricus* strain and to avoid post-acidification while at the same time obtaining high cell counts of the *Lactobacillus delbrueckii* subsp. *bulgaricus* strain, because it would ordinarily have been expected that both the off-flavors and the post-acidification would result from the growth of the *Lactobacillus delbrueckii* subsp. *bulgaricus* strain.

Without being bound by theory it is believed that the surprising effects of the present invention are due to differences in metabolism of the lactose-deficient *Lactobacillus delbrueckii* subsp. *bulgaricus* of the invention as compared to conventional lactose-positive *Lactobacillus delbrueckii* subsp. *bulgaricus.*

### BRI EF DESCRI PTI ON OF THE DRAWINGS

FIGURE 1 is a schematic representation of lactose catabolism in *Streptococcus thermophilus.* GlcK, glucokinase; LacS, lactose transporter; LacZ, β-galactosidase; GalM, mutarotase; GalK, galactokinase; GalT, galactose-1-phosphate uridyltransferase; GalE, UDP-glucose 4 epimerase; Gal1P, galactose-1-phosphate.

### DETAI LED DI SCLOSURE OF THE I NVENTI ON

### Definitions

As used herein, the term "lactic acid bacterium" designates a gram-positive, microaerophilic or anaerobic bacterium, which ferments sugars with the production of acids including lactic acid as the predominantly produced acid, acetic acid and propionic acid. The industrially most useful lactic acid bacteria are found within the order "Lactobacillales" which includes *Lactococcus spp., Streptococcus spp., Lactobacillus spp., Leuconostoc spp., Pediococcus spp., Brevibacterium spp., Enterococcus spp.* and *Propionibacterium spp.* Lactic acid bacteria, including bacteria of the species *Lactobacillus sp.* and *Streptococcus thermophilus,* are normally supplied to the dairy industry either as frozen or freeze-dried cultures for bulk starter propagation or as so-called "Direct Vat Set" (DVS) cultures, intended for direct inoculation into a fermentation vessel or vat for the production of a dairy product, such as a fermented milk product. Such cultures are in general referred to as "starter cultures" or "starters".

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

In connection with the present invention, the expression "increasing the sweetness" means an increase in sweetness as compared to the sweetness produced by a mother strain not carrying a mutation in the DNA sequence of the *glcK* gene encoding a glucokinase protein, wherein the mutation inactivates the glucokinase protein or has a negative effect on expression of the gene.

The expression "CFU" means Colony Forming Units.

### Streptococcus thermophilus strains of the com position of the invention

The present invention does not envisage any mutant strain of *Streptococcus thermophilus* as such.

In some countries, the legal definition of yoghurt requires the presence of both *Streptococcus thermophilus* and *Lactobacillus delbrueckii* subsp. *bulgaricus.* Both species generate desirable amounts of acetaldehyde, an important flavor component in yoghurt.

Cheese, such as Mozzarella and Pizza cheese as well as Feta, can also be prepared by fermentation using both *Streptococcus thermophilus* and *Lactobacillus delbrueckii* subsp. *bulgaricus* (Høier et al. (2010) in The Technology of Cheese making, 2nd Ed. Blackwell Publishing, Oxford; 166-192).

In order to meet the requirements of the food industry, it has become desirable to develop new strains, in particular *Lactobacillus delbruckii* subsp. *bulgaricus* strains and *Streptococcus thermophilus* strains, which produce more natural sweetness directly in the fermented product (inner sweetness) without contributing extra calories.

*Streptococcus thermophilus* is one of the most widely used lactic acid bacteria for commercial milk fermentation where the organism is normally used as part of a mixed starter culture, the other component being a *Lactobacillus* sp., e.g. *Lactobacillus delbrueckii* subsp. *bulgaricus* for yoghurt and *Lactobacillus helveticus* for Swiss-type cheese.

Only the glucose portion of the lactose molecule is fermented by *Streptococcus thermophilus* and galactose accumulates in fermented milk products when *Streptococcus thermophilus* is used. In yoghurt, wherein high acid concentrations limit the fermentation, free galactose remains while the free galactose produced in the early stages of Swiss cheese manufacture is later fermented by *Lactobacillus helveticus. Lactococcus lactis* found in many starter cultures used for cheese manufacture is also capable of consuming the galactose produced by *Streptococcus thermophilus.*

In order to ensure *Streptococcus thermophilus* strains with a growth performance as optimal as possible, the present inventors have exposed galactose-fermenting strains of *Streptococcus thermophilus* to the selective agent 2-deoxyglucose. Typically 2-deoxyglucose resistant mutants have mutations in the gene encoding glucokinase and in genes coding for glucose transport. The isolated mutant, CHCC16731, which are resistant to 2-deoxyglucose have a mutation in its glucokinase (*glcK*) gene. In addition to a mutation in the glucokinase gene, CHCC16731 and CHCC19216 both have a mutation which means that secreted glucose is not transported back into the cells again.

Surprisingly, such mutants alone are still fully capable of acidifying milk although acidification time to pH 5 is delayed by 2-5 hours. They are therefore as such useful in fermented milk applications and they have preserved the ability of the mother strains to acidify the milk which is characteristic of yoghurt. Additionally, it was found that the mutants excreted a high level of glucose, when 9.5% B-milk with 0.05% sucrose was inoculated with the mutants and fermented at 40°C for at least 20 hours. At the same time, very low levels of lactose remain in the fermented milk. Therefore, the use of such strains for producing fermented milk products may have an importance for people with lactose intolerance.

Consequently, the final fermented milk has an increased inner sweetness index calculated as described by Godshall (1988. Food Technology 42(11):71-78).

According to the present invention, the *Streptococcus thermophilus* strain is a galactose-fermenting mutant strain of *Streptococcus thermophilus,* wherein the mutant strain carries a mutation in the DNA sequence of the *glcK* gene encoding a glucokinase protein, wherein the mutation inactivates the encoded glucokinase protein or has a negative effect on expression of the gene, wherein the galactose-fermenting capacity is determined as disclosed in the description and wherein the mutation reduces the activity of the glucokinase protein with at least 50% and wherein the glucokinase activity is determined as indicated in the description.

Methods for measuring the level of glucokinase activity or the level of expression of the glucokinase gene are readily known (Porter et al. (1982) Biochim. Biophys. Acta, 709;178-186) and include enzyme assays with commercially available kits and transcriptomics or quantitative PCR using materials which are readily available.

In a preferred embodiment of the invention, the *Streptococcus thermophilus* strain employed in the composition of the invention is 2-deoxyglucose resistant.

A bacterial "strain" as used herein refers to a bacterium which remains genetically unchanged when grown or multiplied. A multiplicity of identical bacteria is included.

The term "galactose-fermenting *Streptococcus thermophilus* strains" as used herein refers to *Streptococcus thermophilus* strains which are capable of growth on/in M17 medium+2% galactose. The galactose-fermenting *Streptococcus thermophilus* strains are defined herein as *Streptococcus thermophilus* strains which lower the pH of M17 broth containing 2% galactose as sole carbohydrate to 5.5 or lower when inoculated from an overnight culture at 1% and incubated for 24 hours at 37°C.

The term "the mutation inactivates the glucokinase protein" as used herein refers to a mutation which results in an "inactivated glucokinase protein", a glucokinase protein which, if present in a cell, is not able to exert its normal function as well as mutations which prevent the formation of the glucokinase protein or result in degradation of the glucokinase protein.

In particular, an inactivated glucokinase protein is a protein which compared to a functional glucokinase protein is not able to facilitate phosphorylation of glucose to glucose-6-phosphate or facilitates phosphorylation of glucose to glucose-6-phosphate at a significantly reduced rate. The gene encoding such an inactivated glucokinase protein compared to the gene encoding a functional glucokinase protein comprises a mutation in the open reading frame (ORF) of the gene, wherein said mutation may include, but is not limited to, a deletion, a frameshift mutation, introduction of a stop codon or a mutation which results in an amino acid substitution, which changes the functional properties of the protein, or a promoter mutation that reduces or abolishes transcription or translation of the gene.

The mutation reduces the activity (the rate of phosphorylation of glucose to glucose-6-phosphate) of the glucokinase protein with at least 50%, such as at least 60%, such as at least 70%, such as at least 80%, such as at least 90%.

The glucokinase activity is determined by the glucokinase enzymatic assays as described by Pool et al. (2006. Metabolic Engineering 8;456-464).

The term "functional glucokinase protein" as used herein refers to a glucokinase protein which, if present in a cell, facilitates phosphorylation of glucose to glucose-6-phosphate. In particular, a functional glucokinase protein may be encoded by a gene comprising an ORF which has a sequence corresponding to position 1-966 in SEQ ID NO. 1 or a sequence which has at least 85% identity, such as at least 90% identity, such as at least 95% identity, such as at least 98% identity, such as at least 99% identity, to the sequence corresponding to position 1-966 of SEQ ID NO. 1.

The percent identity of two sequences can be determined by using mathematical algorithms, such as the algorithm of Karlin and Altschul (1990. Proc. Natl. Acad. Sci. USA 87;2264), the modified algorithm described in Karlin and Altschul (1993. Proc. Natl. Acad. Sci. USA 90;5873-5877); the algorithm of Myers and Miller (1988. CABIOS 4;11-17); the algorithm of Needleman and Wunsch (1970. J. Mol. Biol. 48;443-453); and algorithm of Pearson and Lipman (1988. Proc. Natl. Acad. Sci. USA 85;2444-2448). Computer software for the determination of nucleic acid or amino acid sequence identity based on these mathematical algorithms is also available. For example, the comparison of nucleotide sequences can be performed with the BLASTN program, score=100, wordlength=12. The comparison of amino acid sequences can be performed with the BLASTX program, score=50, wordlength=3. For the remaining parameters of the BLAST programs, the default parameters can be used.

In many countries the use of genetically modified organisms (GMOs) for fermented milk products is not accepted. The present invention instead provides for compositions comprising naturally occurring or induced mutant strains which can provide a desirable accumulation of glucose in the fermented milk product.

Thus, in a much preferred embodiment of the present invention the composition comprises a mutant strain which is a naturally occurring mutant or an induced mutant.

A "mutant bacterium" or a "mutant strain" as used herein refers to a natural (spontaneous, naturally occurring) mutant bacterium or an induced mutant bacterium comprising one or more mutations in its genome (DNA) which are absent in the wild type DNA. An "induced mutant" is a bacterium where the mutation was induced by human treatment, such as treatment with chemical mutagens, UV- or gamma radiation etc. In contrast, a "spontaneous mutant" or "naturally occurring mutant" has not been mutagenized by man. Mutant bacteria are herein, non-GMO (non-genetically modified organism), i.e. not modified by recombinant DNA technology.

"Wild type strain" refers to the non-mutated form of a bacterium, as found in nature.

Terms such as "strains with a sweetening property", "strains which can provide a desirable accumulation of glucose in the fermented milk product" and "strains with enhanced properties for natural sweetening of food products" are used interchangeably herein to characterize an advantageous aspect of using the strains employed in the present invention in fermentation of milk products.

In a preferred embodiment, the mutant strain of *Streptococcus thermophilus* employed in the invention increases the amount of glucose in 9.5% B-milk to at least 5 mg/mL when inoculated into the 9.5% B-milk at a concentration of 10⁶-10⁷ CFU/ml and grown at 40°C for at least 20 hours.

In another preferred embodiment, the mutant strain of *Streptococcus thermophilus* employed in the invention increases the amount of glucose in 9.5% B-milk with 0.05% sucrose to at least 5 mg/mL when inoculated into the 9.5% B-milk with 0.05% sucrose at a concentration of 10⁶-10⁷ CFU/ml and grown at 40°C for at least 20 hours.

In the present context, 9.5% B-milk is boiled milk made with reconstituted low fat skim milk powder to a level of dry matter of 9.5% and pasteurized at 99°C for 30 min. followed by cooling to 40°C.

In more preferred embodiments of the invention the mutant strain employed in the invention leads to an increase in the amount of glucose to at least 6 mg/mL, such as at least 7 mg/mL, such as at least 8 mg/mL, such as at least 9 mg/ml, such as at least 10 mg/ml, such as at least 11 mg/ml, such as at least 12 mg/ml, such as at least 13 mg/ml, such as at least 14 mg/ml, such as at least 15 mg/ml, such as at least 20 mg/ml, such as at least 25 mg/ml.

In another embodiment of the invention the mutant strain of *Streptococcus thermophilus* employed in the invention is resistant to 2-deoxyglucose. The term "resistant to 2-deoxyglucose" herein is defined by that a particular mutated bacterial strain has the ability to grow to a colony when streaked on a plate of M17 medium containing 20 mM 2-deoxyglucose after incubation at 40°C for 20 hours. The presence of 2-deoxygluxcose in the culture medium will prevent the growth of non-mutated strains while the growth of the mutated strains is not affected or not affected significantly. Non-mutated strains which can be used as sensitive reference strains in the assessment of resistance preferably include the strains CHCC14994 and CHCC11976.

The mutant *Streptococcus thermophilus* strains employed in the invention excrete glucose into the milk when 9.5% B-milk is inoculated with 10⁶-10⁷ CFU/ml of a *Streptococcus thermophilus* strain according to the invention and fermented with the *Streptococcus thermophilus* strains according to the invention at 40°C for at least 20 hours. Preferably, such mutant strains alone will excrete at least 5 mg/ml glucose into B-milk when 9.5% B-milk is inoculated with 10⁶-10⁷ CFU/ml of a *Streptococcus thermophilus* strain according to the invention and fermented with the *Streptococcus thermophilus* strains at 40°C for at least 20 hours. The strains are still fully capable of acidifying milk although acidification time to pH 5 is delayed by 2-5 hours. The final fermented milk contains less than 15 mg/ml lactose in the fermented milk. The final fermented milk consequently has a higher inner sweetness index of approximately 2 fold or more.

In yet another embodiment, the mutant strain according to the invention can be characterized by its growth pattern. This is illustrated by the finding that the growth rate of the mutant strain is higher in M17 medium+2% galactose than in M17 medium+2% glucose. The growth rate is measured as the development in optical density of the exponentially growing culture at 600 nanometers (OD₆₀₀) with time.

In a preferred embodiment the growth rate is at least 5% higher, such as at least 10% higher, such as at least 15% higher, such as at least 20% higher, in M17 medium+2% galactose than in M17 medium+2% glucose.

### Mutation in glcK gene

In one preferred embodiment the mutation results in the replacement of the codon coding for glycine with the codon coding for arginine in position 249 in SEQ ID NO. 2. Preferably the mutation in the *glcK* gene results in the replacement of a G with a A in position 745 in SEQ ID NO. 1. Strain CHCC16731 has such a mutation.

In a preferred embodiment the mutation results in the replacement of the codon coding for serine with the codon coding for proline in position 72 in SEQ ID NO. 2 (not shown). Preferably the mutation in the *glcK* gene results in the replacement of a T with a C in position 214 in SEQ ID NO. 1 (not shown).

In another preferred embodiment the mutation results in the replacement of the codon coding for threonine with the codon coding for isoleucine in position 141 in SEQ ID NO. 2 (not shown).

Preferably, the mutation in the *glcK* gene results in the replacement of a C with a T on position 422 in SEQ ID NO. 1 (not shown).

It should be emphasized that the *glcK* gene of a *Streptococcus thermophilus* may be inactivated by other types of mutations in other sites of the *glcK* gene.

### Mutation reducing transport of glucose into the cell

In a preferred embodiment the *Streptococcus thermophilus* strain carries a mutation that reduces the transport of glucose into the cell.

The term "a mutation that reduces the transport of glucose into the cell" as used herein refers to a mutation in a gene encoding a protein involved in transport of glucose which results in an accumulation of glucose in the environment of the cell.

The level of glucose in the culture medium of a *Streptococcus thermophilus* strain can readily be measured by methods known to the skilled person also when the culture medium is a milk substrate.

In preferred embodiments the mutation reduces the transport of glucose into the cell with at least 50%, such as at least 60%, such as at least 70%, such as at least 80%, such as at least 90%.

The transport of glucose into the cell can be determined by the glucose uptake assay as described by Cochu et al. (2003. Appl Environ Microbiol 69(9); 5423-5432).

Preferably, the *Streptococcus thermophilus* strain carries a mutation in a gene encoding a component of a glucose transporter, wherein the mutation inactivates the glucose transporter protein or has a negative effect on expression of the gene.

The component may be any component of a glucose transporter protein which is critical for the transport of glucose. It is e.g. contemplated that inactivation of any component of the glucose/mannose PTS in *Streptococcus thermophilus* will result in inactivation of the glucose transporter function.

The term "the mutation inactivates the glucose transporter" as used herein refers to a mutation which results in an "inactivated glucose transporter", a glucose transporter protein which, if present in a cell, is not able to exert its normal function as well as mutations which prevent the formation of the glucose transporter protein or result in degradation of the glucose transporter protein.

In particular, an inactivated glucose transporter protein is a protein which compared to a functional glucose transporter protein is not able to facilitate transport of glucose over a plasma membrane or facilitates transport of glucose over a plasma membrane at a significantly reduced rate. The gene encoding such an inactivated glucose transporter protein compared to the gene encoding a functional glucose transporter protein comprises a mutation in the open reading frame (ORF) of the gene, wherein said mutation may include, but is not limited to, a deletion, a frameshift mutation, introduction of a stop codon or a mutation which results in an amino acid substitution, which changes the functional properties of the protein, or a promoter mutation that reduces or abolishes transcription or translation of the gene.

In preferred embodiments the mutation reduces the activity (the rate of transport of glucose) of the glucose transporter protein by at least 50%, such as at least 60%, such as at least 70%, such as at least 80%, such as at least 90%.

The glucose transporter activity can be determined by the glucose uptake assay as described by Cochu et al. (2003. Appl Environ Microbiol 69(9); 5423-5432).

The term "functional glucose transporter protein" as used herein refers to a glucose transporter protein which, if present in a cell, facilitates transport of glucose over a plasma membrane.

In a preferred embodiment of the invention the *Streptococcus thermophilus* strain employed in the invention carries a mutation in the DNA sequence of the *manN* gene encoding the IID^{Man} protein of the glucose/mannose phosphotransferase system, wherein the mutation inactivates the IID^{Man} protein or has a negative effect on expression of the gene.

CHCC16731 has a threonine to proline change at position 79 in the *manN* gene encoding the IID^{Man} protein of the glucose/mannose phosphotransferase system. Preferably the mutation in the *ManN* gene results in the replacement of an A with a C in position 235 in SEQ ID NO. 3.

Thus, in a preferred embodiment the *Streptococcus thermophilus* strain employed in the invention carries a mutation in the DNA sequence of the *manN* gene encoding the IID^{Man} protein of the glucose/mannose phosphotransferase system, wherein the mutation results in the replacement of threonine to proline in position 79 of SEQ ID No. 4.

In another preferred embodiment of the invention the *Streptococcus thermophilus* strain employed in the invention carries a mutation in the DNA sequence of the *manM* gene encoding the IIC^{Man} protein of the glucose/mannose phosphotransferase system, wherein the mutation inactivates the IIC^{Man} protein or has a negative effect on expression of the gene.

In a specific preferred embodiment the mutation results in the replacement of the codon coding for glutamic acid with a stop codon in position 209 of SEQ ID NO. 6 of the IIC^{Man} protein of the glucose/mannose phosphotransferase system (not shown). Preferably, the mutation results in the replacement of a G with a T in position 625 of SEQ ID NO. 5 (not shown).

### Preferred Streptococcus thermophilus strains employed in compositions of the invention

In a preferred embodiment of the composition of the invention the *Streptococcus thermophilus* strain is selected from the group consisting of the *Streptococcus thermophilus* CHCC19216 strain that was deposited at Deutsche Sammlung von Mikroorganismen und Zellkulturen under accession No. DSM 32227, the strain *Streptococcus thermophilus* CHCC16731 has been deposited at Deutsche Sammlung von Mikroorganismen und Zellkulturen (DSMZ) GmbH, Inhoffenstr. 7B, D-38124 Braunschweig, Germany, on 4 June 2014 under the accession No. DSM 28889, the *Streptococcus thermophilus* CHCC15757 strain that was deposited at Deutsche Sammlung von Mikroorganismen und Zellkulturen under accession No. DSM 25850, a *Streptococcus thermophilus* CHCC15887 strain that was deposited at Deutsche Sammlung von Mikroorganismen und Zellkulturen under accession No. DSM 25851, the *Streptococcus thermophilus* CHCC16404 strain that was deposited at Deutsche Sammlung von Mikroorganismen und Zellkulturen under accession No. DSM 26722, and a mutant strain derived therefrom, wherein the mutant strain is obtained by using one of the deposited strains as starting material, and wherein the mutant has retained or further improved the texturizing property and the glucose secreting property of said deposited strain.

In the present context, the term "mutant strain" should be understood as strains derived from their mother strains employed in the invention by means of e.g. genetic engineering, radiation and/or chemical treatment. The "strains derived therefrom" can also be spontaneously occurring mutants. The "strains derived therefrom" are functionally equivalent mutants, i.e. mutants that have substantially the same, or improved properties as their mother strain. Especially, the term "mutant strains" refers to strains obtained by subjecting a strain employed in the invention to any conventionally used mutagenization treatment including treatment with a chemical mutagen such as ethane methane sulphonate (EMS) or N-methyl-N'-nitro-N-nitroguanidine (NTG), UV light, or to a spontaneously occurring mutant. A mutant may have been subjected to several mutagenization treatments (a single treatment should be understood as one mutagenization step followed by a screening/selection step), but it is presently preferred that no more than 20, or no more than 10, or no more than 5, treatments (or screening/selection steps) are carried out. In a mutant less than 1%, less than 0.1%, less than 0.01%, less than 0.001% or even less than 0.0001% of the nucleotides in the bacterial genome have been replaced with another nucleotide, or deleted, compared to the mother strain.

### Method of obtaining a sweetening Streptococcus thermophilus strain

Methods of obtaining sweetening strains of *Streptococcus thermophilus* are not embraced by the present invention and present for illustrative purposes only.

The starting strain used for developing a sweetening *Streptococcus thermophilus* strain is a texturizing *Streptococcus thermophilus* strain, which may be obtained as described elsewhere in this specification.

In a preferred embodiment such a texturizing starting strain is selected from the group consisting of the *Streptococcus thermophilus* CHCC11342 strain that was deposited at Deutsche Sammlung von Mikroorganismen und Zellkulturen under accession No. DSM 22932, the *Streptococcus thermophilus* CHCC11976 strain that was deposited at Deutsche Sammlung von Mikroorganismen und Zellkulturen under accession No. DSM 22934, the *Streptococcus thermophilus* CHCC12339 strain that was deposited at Deutsche Sammlung von Mikroorganismen und Zellkulturen under accession No. DSM 24090, and strains derived therefrom.

Alternatively, the starting strain used for developing a sweetening *Streptococcus thermophilus* strain is a non-texturizing *Streptococcus thermophilus* strain, in which case the texturizing property is introduced into the sweetening *Streptococcus thermophilus* strain once produced, i.e. the sweetening strain is used as a starting strain in the method of obtaining a texturizing strain described elsewhere in this specification.

The first step of the method of obtaining a sweetening *Streptococcus thermophilus* strain is to provide a galactose positive strain, i.e. a strain that is capable of using galactose as a carbohydrate source. Galactose positive strains may be obtained by a method comprising the step of streaking the bacteria to be tested on agar plates, such as M17 agar plates containing a certain concentration, such as 2 %, of galactose (sole carbohydrate source) and identifying colonies able to grow on the plates.

2-deoxyglucose and a determination of the growth pattern of the bacteria in M17 medium+2% galactose compared to in M17 medium+2% glucose is used for the selection of bacteria having a mutation in the glucokinase (*glcK*) gene.

One method for screening and isolating a strain of *Streptococcus thermophilus* with a mutated *glcK* gene comprises the following steps:
a) providing a galactose-fermenting *Streptococcus thermophilus* mother strain;
b) selecting and isolating from a pool of mutant *Streptococcus thermophilus* strains derived from the mother strain a pool of mutant *Streptococcus thermophilus* strains which are resistant to 2-deoxyglucose; and
c) selecting and isolating from the pool of mutant *Streptococcus thermophilus* strains which are resistant to 2-deoxyglucose a mutant *Streptococcus thermophilus* strain if the growth rate of the mutant *Streptococcus thermophilus* strain is higher in M17 medium+2% galactose than in M17 medium+2% glucose.

The term "resistant to 2-deoxyglucose" herein is defined by that a particular mutated bacterial strain has the ability to grow to a colony when streaked on a plate of M17 medium containing 2% lactose or 2% galactose and containing 20 mM 2-deoxyglucose after incubation at 40°C for 20 hours. The presence of 2-deoxygluxcose in the culture medium will prevent the growth of non-mutated strains while the growth of the mutated strains is not affected or not affected significantly. Non-mutated strains which can be used as sensitive reference strains in the assessment of resistance include the strain CHCC11976.

In an embodiment the method further comprises the step a1) subjecting the mother strain to mutagenization, such as subjecting the mother strain to a chemical and/or a physical mutagen.

In another embodiment the method further comprises a step d) selecting and isolating from a pool of 2-deoxyglucose resistant *Streptococcus thermophilus* strains derived from the *Streptococcus thermophilus* strain selected in step c) a *Streptococcus thermophilus* strain if the growth rate of the Streptococcus thermophilus strain is high in M17 medium+2% sucrose but zero or at least 0-50% reduced compared to the growth rate of the mother strain in M17 medium+2% glucose.

The galactose-fermenting *Streptococcus thermophilus* mother strains are capable of growth on/in M17 medium+2% galactose and are defined herein by that they have the ability to lower the pH in M17 broth containing 2% galactose as sole carbohydrate to 5.5 or lower when inoculated from an overnight culture at 1% and incubated for 24 hours at 37°C. Such galactose-positive strains have been described in WO2011/026863 (Chr. Hansen A/S) and WO2011/092300 (Chr. Hansen A/S).

In the present context, the term "strains derived therefrom" should be understood as strains derived, or strains which can be derived from texturizing or galactose-fermenting *Streptococcus thermophilus* mother strains by means of e.g. genetic engineering, radiation and/or chemical treatment. The "strains derived therefrom" can also be spontaneously occurring mutants. The "strains derived therefrom" are functionally equivalent mutants, i.e. mutants that have substantially the same, or improved properties (e.g. regarding texture or fermentation of galactose) as their mother strain. Especially, the term "strains derived therefrom" refers to strains obtained by subjecting a strain of the invention to any conventionally used mutagenization treatment including treatment with a chemical mutagen such as ethane methane sulphonate (EMS) or N-methyl-N'-nitro-N-nitroguanidine (NTG), UV light, or to a spontaneously occurring mutant. A mutant may have been subjected to several mutagenization treatments (a single treatment should be understood one mutagenization step followed by a screening/selection step), but it is presently preferred that no more than 20, or no more than 10, or no more than 5, treatments (or screening/selection steps) are carried out. In a mutant less than 1%, less than 0.1%, less than 0.01%, less than 0.001% or even less than 0.0001% of the nucleotides in the bacterial genome have been replaced with another nucleotide, or deleted, compared to the mother strain.

In the present invention the expression *"glcK* gene encoding a glucokinase" means any DNA sequence of a *Streptococcus thermophilus* encoding a protein having glucokinase activity, including the specific glucokinase encoded by the DNA sequence of SEQ ID NO.: 1. A glucokinase catalyzes the reaction converting glucose to glucose-6-phosphate, cf. Fig. 1.

### Lactobacillus delbrueckii subsp. bulgaricus strains of the composition of the invention

The present invention does not envisage any mutant strain of *Lactobacillus delbrueckii subsp. bulgaricus* as such.

The terms "deficiency in lactose metabolism" and "lactose deficient" are used in the context of the present invention to characterize LAB which either partially or completely lost the ability to use lactose as a source for cell growth or maintaining cell viability. Respective LAB are capable of metabolizing glucose.

Since this carbohydrate is not naturally present in milk in sufficient amounts to support fermentation by lactose-deficient mutants, it is necessary to add it to the milk.

Lactose deficient and partially deficient LAB can be characterized as white colonies on a medium containing lactose and X-Gal.

In connection with the present invention the term "X-Gal" means 5-bromo-4-chloro-3-indolyl-beta-D-galacto-pyranoside, which is a chromogenic substrate for beta-galactosidase, which hydrolyses X-Gal into colorless galactose and 5-bromo-4-chloro-indoxyl, which spontaneously dimerizes to form a blue pigment.

The lactose-deficient strain is capable of metabolizing glucose.

In a particular embodiment of the composition of the invention, the Lb strain is selected from the group consisting of the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28910, and a mutant strain derived from DSM 28910, wherein the mutant strain is further characterized as having the ability to generate white colonies on a medium containing lactose and X-Gal.

Lactose deficient strains may be obtained from a suitable lactose-positive mother strain by mutation. The lactose-deficient strains were selected after UV-mutagenesis as white colonies (indicating a lactose deficient phenotype) on a suitable medium, e.g. MRS agar plates with 1% lactose and 200 mg/ml X-Gal. Lactose-positive strains possesses β-galactosidase activity, and colonies of lacrosse-positive strain appear blue due to the activity of the β-galactosidase. As lactose-positive mother strain for producing a lactose-deficient strain, the *Lactobacillus delbrueckii* ssp. *bulgaricus* strain CHCC10019 deposited for WO2011/000879 with DSMZ-Deutsche Sammlung von Mikroorganismen und Zellkulturen GmbH, Inhoffenstr. 7B, D-38124 Braunschweig on 2007-04-03 under the accession No. DSM 19252, may be used.

### Com position

The present invention further relates to a compositions as defined in claim 1 comprising from 10⁴ to 10¹² CFU (colony forming units)/g of the *Streptococcus thermophilus* strain, such as from 10⁵ to 10¹¹ CFU/g, such as from 10⁶ to 10¹⁰ CFU/g, or such as from 10⁷ to 109 CFU/g of the *Streptococcus thermophilus* strain.

In a preferred embodiment the *Streptococcus thermophilus* strain of the composition is unable to acidify 9.5% B-milk, defined as resulting in a pH decrease of less than 1.0 when 9.5% B-milk is inoculated with 10⁶-10⁷ CFU/ml of the *Streptococcus thermophilus* strain and incubated for 14 hours at 40°C, and the composition further comprises an amount of a compound, which can trigger acidification of the 9.5% B-milk by the *Streptococcus thermophilus* strain CHCC16404 that was deposited at Deutsche Sammlung von Mikroorganismen und Zellkulturen under accession No. DSM 26722, defined as resulting in a pH decrease of 1.0 or more when 9.5% B-milk is inoculated with 10⁶-10⁷ CFU/ml of the *Streptococcus thermophilus* strain and incubated for 14 hours at 40°C.

Preferably, the compound is sucrose.

Preferably, the amount of sucrose is from 0.000001% to 2%, such as from 0.00001% to 0.2%, such as from 0.0001% to 0.1%, such as from 0.001% to 0.05%.

The composition as set forth in claim 1 preferably further comprises from 10⁴ to 10¹² CFU/g of the *Lactobacillus delbrueckii* subsp. *bulgaricus* strain, such as from 10⁵ to 10¹¹ CFU/g, such as from 10⁶ to 10¹⁰ CFU/g, or such as from 10⁷ to 10⁹ CFU/g of the *Lactobacillus delbrueckii* subsp. *bulgaricus* strain.

In a particular embodiment of the invention, the composition according to claim 1 contains at least two St strains, preferably at least three St strains, and more preferably three St strains. In a particular embodiment of the invention, the composition according to claim 1 contains no more than three Lb strains, preferably no more than two Lb strains, and more preferably one Lb strain.

In a particular embodiment of the invention, the composition according to claim 1 contains at least one St strain selected from the group consisting of the strain *Streptococcus thermophilus* CHCC16731 has been deposited at Deutsche Sammlung von Mikroorganismen und Zellkulturen (DSMZ) GmbH, Inhoffenstr. 7B, D-38124 Braunschweig, Germany, on 4 June 2014 under the accession No. DSM 28889, the *Streptococcus thermophilus* CHCC15757 strain that was deposited at Deutsche Sammlung von Mikroorganismen und Zellkulturen under accession No. DSM 25850, and the *Streptococcus thermophilus* CHCC16404 strain that was deposited at Deutsche Sammlung von Mikroorganismen und Zellkulturen under accession No. DSM 26722, and the Lb strain deposited with DSMZ-Deutsche Sammlung von Mikroorganismen und Zellkulturen GmbH, Inhoffenstr. 7B, D-38124 Braunschweig, on 2014-06-12 under the accession no. DSM 28910.

*Lactobacillus delbrueckii* subsp. *bulgaricus, Streptococcus thermophilus* and other lactic acid bacteria are commonly used as starter cultures serving a technological purpose in the production of various foods, such as in the dairy industry, such as for fermented milk products. Thus, in another preferred embodiment the composition is suitable as a starter culture.

Starter cultures may be provided as frozen or dried starter cultures in addition to liquid starter cultures. Thus, in yet another preferred embodiment the composition is in frozen, freeze-dried or liquid form.

As disclosed in WO 2005/003327, it is beneficial to add certain cryoprotective agents to a starter culture. Thus, a starter culture composition according to the present invention may comprise one or more cryoprotective agent(s) selected from the group consisting of inosine-5'-monophosphate (IMP), adenosine -5'-monophosphate (AMP), guanosine-5'-monophosphate (GMP), uranosine-5'-monophosphate (UMP), cytidine-5'-monophosphate (CMP), adenine, guanine, uracil, cytosine, adenosine, guanosine, uridine, cytidine, hypoxanthine, xanthine, hypoxanthine, orotidine, thymidine, inosine and a derivative of any such compounds.

### Method of producing a fermented milk product

The invention is further directed to a method of producing a fermented milk product comprising inoculating and fermenting a milk substrate with the composition according to the present invention.

The term "milk" is to be understood as the lacteal secretion obtained by milking any mammal, such as a cow, a sheep, a goat, a buffalo or a camel. In a preferred embodiment, the milk is cow's milk.

The term "milk substrate" may be any raw and/or processed milk material that can be subjected to fermentation according to the method of the invention. Thus, useful milk substrates include, but are not limited to, solutions/suspensions of any milk or milk like products comprising protein, such as whole or low fat milk, skim milk, buttermilk, reconstituted milk powder, condensed milk, dried milk, whey, whey permeate, lactose, mother liquid from crystallization of lactose, whey protein concentrate, or cream. Obviously, the milk substrate may originate from any mammal, e.g. being substantially pure mammalian milk, or reconstituted milk powder.

Preferably, at least part of the protein in the milk substrate is proteins naturally occurring in milk, such as casein or whey protein. However, part of the protein may be proteins which are not naturally occurring in milk.

Prior to fermentation, the milk substrate may be homogenized and pasteurized according to methods known in the art.

"Homogenizing" as used herein means intensive mixing to obtain a soluble suspension or emulsion. If homogenization is performed prior to fermentation, it may be performed so as to break up the milk fat into smaller sizes so that it no longer separates from the milk. This may be accomplished by forcing the milk at high pressure through small orifices.

"Pasteurizing" as used herein means treatment of the milk substrate to reduce or eliminate the presence of live organisms, such as microorganisms. Preferably, pasteurization is attained by maintaining a specified temperature for a specified period of time. The specified temperature is usually attained by heating. The temperature and duration may be selected in order to kill or inactivate certain bacteria, such as harmful bacteria. A rapid cooling step may follow.

"Fermentation" in the methods of the present invention means the conversion of carbohydrates into alcohols or acids through the action of a microorganism. Preferably, fermentation in the methods of the invention comprises conversion of lactose to lactic acid.

Fermentation processes to be used in production of fermented milk products are well known and the person of skill in the art will know how to select suitable process conditions, such as temperature, oxygen, amount and characteristics of microorganism(s) and process time. Obviously, fermentation conditions are selected so as to support the achievement of the present invention, i.e. to obtain a dairy product in solid or liquid form (fermented milk product).

The term "fermented milk product" as used herein refers to a food or feed product wherein the preparation of the food or feed product involves fermentation of a milk substrate with a lactic acid bacteria. "Fermented milk product" as used herein includes but is not limited to products such as thermophilic fermented milk products, e.g. yoghurt, mesophilic fermented milk products, e.g. sour cream and buttermilk, as well as fermented whey.

The term "thermophile" herein refers to microorganisms that thrive best at temperatures above 35°C. The industrially most useful thermophilic bacteria include Streptococcus spp. and Lactobacillus spp. The term "thermophilic fermentation" herein refers to fermentation at a temperature above about 35°C, such as between about 35°C to about 45°C. The term "thermophilic fermented milk product" refers to fermented milk products prepared by thermophilic fermentation of a thermophilic starter culture and include such fermented milk products as set-yoghurt, stirred-yoghurt and drinking yoghurt, e.g. Yakult.

The term "mesophile" herein refers to microorganisms that thrive best at moderate temperatures (15°C-35°C). The industrially most useful mesophilic bacteria include Lactococcus spp. and Leuconostoc spp. The term "mesophilic fermentation" herein refers to fermentation at a temperature between about 22°C and about 35°C. The term "mesophilic fermented milk product" refers to fermented milk products prepared by mesophilic fermentation of a mesophilic starter culture and include such fermented milk products as buttermilk, sour milk, cultured milk, smetana, sour cream, Kefir and fresh cheese, such as quark, tvarog and cream cheese.

In a preferred embodiment the concentration of *Streptococcus thermophilus* cells inoculated is from 10⁴ to 10⁹ CFU *Streptococcus thermophilus* cells per ml of milk substrate, such as from 10⁴ CFU to 10⁸ CFU *Streptococcus thermophilus* cells per ml of milk substrate.

In another preferred embodiment of the method of the invention the *Streptococcus thermophilus* strain is unable to acidify the 9.5% B-milk, defined as resulting in a pH decrease of less than 1.0 when 9.5% B-milk is inoculated with 10⁶-10⁷ CFU/ml of the *Streptococcus thermophilus* strain and incubated for 14 hours at 40°C, and the milk substrate is added an amount of a compound, effective to trigger acidification of 9.5% B-milk by the *Streptococcus thermophilus* strain, defined as resulting in a pH decrease of 1.0 or more when 9.5% B-milk is inoculated with 10⁶-10⁷ CFU/ml of the *Streptococus thermophilus* strain and incubated for 14 hours at 40°C.

Preferably, the compound is sucrose.

Preferably, the amount of sucrose is from 0.000001% to 2%, such as from 0.00001% to 0.2%, such as from 0.0001% to 0.1%, such as from 0.001% to 0.05%.

In another preferred embodiment the fermented milk product is a yoghurt or a cheese.

Examples of cheeses which are prepared by fermentation with *Streptococcus thermophilus* and *Lactobacillus delbrueckii* subsp. *bulgaricus* include Mozzarella and pizza cheese (Høier et al. (2010) in The Technology of Cheesemaking, 2nd Ed. Blackwell Publishing, Oxford; 166-192).

Preferably the fermented milk product is a yoghurt.

In the present context, a yoghurt starter culture is a bacterial culture which comprises at least one *Lactobacillus delbrueckii* subsp. *bulgaricus* strain and at least one *Streptococcus thermophilus* strain. In accordance herewith, the term "yoghurt" refers to a fermented milk product obtainable by inoculating and fermenting milk with a composition comprising a *Lactobacillus delbrueckii* subsp. *bulgaricus* strain and a *Streptococcus thermophilus* strain.

### Fermented milk product

The present invention further relates to a fermented milk product comprising the composition according to the invention.

In another preferred embodiment the fermented milk product may be a yoghurt, a cheese, sour cream and buttermilk as well as fermented whey. Preferably, the fermented milk product is a yoghurt.

### Use of the composition of the invention

A further aspect of the invention relates to the use of the composition according to the invention for the preparation of a fermented milk product.

Embodiments of the present invention are described below, by way of nonlimiting examples.

### Sequence listing

- SEQ ID NO. 1: shows the DNA sequence of the mutated glucokinase gene of strain CHCC16731.
- SEQ ID NO. 2: shows the amino acid sequence encoded by SEQ ID NO. 1.
- SEQ ID NO. 3: shows the DNA sequence of the mutated *ManN* gene of strain CHCC16731.
- SEQ ID NO. 4: shows the amino acid sequence encoded by SEQ ID NO. 3.
- SEQ ID NO. 5: shows the DNA sequence of the *ManM* gene (not mutated) of strain CHCC16731.
- SEQ ID NO. 6: shows the amino acid sequence encoded by SEQ ID NO. 5.

### EXAMPLES

### Materials and methods

### Medium:

For *Streptococcus thermophilus,* the medium used is the M17 medium known to persons skilled in the art.

The M17 agar medium has the following composition per liter H₂O:
agar, 12.75 g
ascorbic acid, 0.5 g
casein peptone (tryptic), 2.5 g
disodium β-glycerophosphate pentahydrate, 19 g
magnesium sulfate hydrate, 0.25 g
meat extract, 5 g
meat peptone (peptic), 2.5 g
soyapeptone (papainic), 5 g
yeast extract, 2.5 g
final pH 7.1±0.2 (25°C)
and M17 broth has the following composition per liter H₂O:
   ascorbic acid, 0.5 g
   magnesium sulfate, 0.25 g
   meat extract, 5 g
   meat peptone (peptic), 2.5 g
   sodium glycerophosphate, 19 g
   soya peptone (papainic), 5 g
   tryptone, 2.5 g
   yeast extract, 2.5 g
   final pH 7.0±0.2 (25°C)
   Carbon sources added are sterile lactose 20 g/l, glucose 20 g/l or galactose 20 g/l.

As known to the skilled person, the M17 medium is a medium that is considered to be suitable for growth of *Streptococcus thermophilus.* Further, as understood by the skilled person, in the present context, a M17 concentrate may be supplied from different suppliers and independently of the specific supplier one will (within standard measurement uncertainty) get the same herein relevant result of 2-deoxyglucose resistance for a herein relevant cell of interest.

The medium used for culturing *Lactobacillus delbrueckii* subsp. *bulgaricus* was MRS-IM medium. MRS-IM was used either in the form of agar plates or broth.

The MRS-IM agar medium had the following composition per liter H₂O:

| | | |
|---|---|---|
| Tryptone | Oxoid L 42 | 10.0 g |
| Yeast extract | Oxoid L 21 | 5.0 g |
| Tween 80 | Merck nr 8.22187 | 1.0 g |
| K₂HPO₄ | Merck nr 105104 | 2.6 g |
| Na-acetate | Merck nr 106267 | 5.0 g |
| Diammonium-hydrogen-citrate | Merck nr 101154 | 2.0 g |
| MgSO₄, 7 H2O | Merck nr 105882 | 0.2 g |
| MnSO₄, H2O | Merck nr 105941 | 0.05 g |
| Agar | SO-BI-GEL | 13.0 g |

The pH was adjusted after autoclaving to 6.9 ± 0.1 at 25°C.

The MRS-IM broth used in the below examples for liquid cultures had the following composition per liter H₂O:

| | | |
|---|---|---|
| Tryptone | Oxoid L 42 | 10.0 g |
| Yeast extract | Oxoid L 21 | 5.0 g |
| Tween 80 | Merck nr 8.22187 | 1.0 g |
| K₂HPO₄ | Merck nr 105104 | 2.6 g |
| Na-acetate | Merck nr 106267 | 5.0 g |
| Diammonium-hydrogen-citrate | Merck nr 101154 | 2.0 g |
| MgSO₄, 7 H2O | Merck nr 105882 | 0.2 g |
| MnSO₄, H2O | Merck nr 105941 | 0.05 g |

The pH is adjusted after autoclaving to 6.9 ± 0.1 at 25°C. The carbon sources, lactose 20g/l or glucose 20g/l, were first filtered sterile and then added to the autoclaved broth.

The above MRS-IM media can be varied to some extent without affecting the capability of the media to support growth of *Lactobacillus delbrueckii* subsp. *bulgaricus.* Further, as will be understood by the skilled person, a MRS-IM concentrate or the various components described above may be obtained from different suppliers and used for the preparation of a MRS-IM medium. These media will likewise be used in the below examples, in particular in the 2-deoxyglucose resistance selection assay.

### Mother strains

*Streptococcus thermophilus* CHCC11976 (galactose-fermenting strain with a mutation in the *GalK* gene as described in WO 2011/026863).

*Streptococcus thermophilus* CHCC12339 (phage resistant and texturizing as described in WO2011/092300).

*Streptococcus thermophilus* CHCC18948 (galactose-fermenting mutant of CGCC12339 with a mutation in the *GalK* gene)

### 2-deoxy-glucose resistant strains

*Streptococcus thermophilus* CHCC16165 (2-deoxyglucose resistant mutant of CHCC11976).

*Streptococcus thermophilus* CHCC16731 (sweetening and texturizing mutant of CHCC16165).

*Streptococcus thermophilus* CHCC19216 (sweetening and texturizing mutant of CHCC18948).

### EXAMPLE 1: Use of 2-deoxyglucose to isolate glucose kinase mutant of Streptococcus thermophilus CHCC1 1976 with enhanced excretion of glucose (Example not according to the invention and presented for illustrative purposes only)

In order to isolate mutants of *Streptococcus thermophilus* strain CHCC11976, cells derived from the growth of a single colony were inoculated into 10 ml of M17 broth containing 2% lactose and grown overnight at 40°C.

Next day, the strains were plated in serial dilutions on M17 agar plates containing 2% galactose and a concentration of 2-deoxyglucose of 20 mM (CHCC11976) and incubated for 20 hours at 40°C. Resistant colonies were at first re-streaked on the same type of agar plates as they were selected. Survivors were used to inoculate fresh M17 broth containing either 2% lactose, 2% galactose or 2% glucose and growth was measured.

From this, a number of mutants that were able to grow faster on galactose than on glucose were identified as outlined in Example 2. One such mutant was CHCC16165.

### EXAMPLE 2: 2-deoxyglucose resistance mutant growth pattern (Example not according to the invention and presented for illustrative purposes only)

To ensure the selection of 2-deoxyglucose resistant mutants that can grow on galactose, two strains that were selected from a galactose-fermenting strain collection were used. While these galactose-fermenting strains still grow at least 10% faster in exponential phase in M17 broth+2% glucose than in M17 broth+2% galactose, the 2-deoxyglucose resistant mutant derivative of CHCC11976, i.a. CHCC16165, on the other hand, are characterized by growing faster in exponential phase in M17 broth+2% galactose than in M17 broth+2% glucose.

Growth in exponential phase is herein measured as the development in optical density of the exponentially growing culture at 600 nanometers (OD₆₀₀) with time at 40°C.

As known by the skilled person, it may vary from species to species when the culture is in exponential growth. The skilled person will know how to determine the growth in exponential phase, e.g. between OD₆₀₀ 0.1-1.0.

The optical density (OD) of the culture is measured in a spectrophotometer.

### Conclusion:

Based on the 2-deoxyglucose resistance mutant growth pattern of this Example 2
- for a specific strain of interest (e.g. one from a relevant commercial product) - the skilled person can routinely test if this specific strain of interest has the herein relevant growth pattern which is a property of the selected mutants.

### EXAMPLE 3: Selection of a hyper-lactose fermenting and glucose secreting mutant of Streptococcus thermophilus CHCC16165

In order to isolate a hyper-lactose fermenting and glucose secreting mutant of *Streptococcus thermophilus* strain CHCC16165, cells derived from the growth of a single colony were inoculated into 10 ml of M17 broth containing 2% galactose and grown overnight at 40°C.

Next day, the strain was plated in serial dilutions on M17 agar plates containing 2% sucrose and a concentration of 2-deoxyglucose of 40 mM and incubated for 20 hours at 40°C. 10 random colonies were picked from the plate and used to inoculate fresh M17 broth containing 2% sucrose and incubated over night at 40°C. The CHCC16165 mutants were transferred to fresh M17 media containing 2% sucrose.

### EXAMPLE 4. Carbohydrate analysis of fermented milk. (Example not according to the invention and presented for illustrative purposes only)

The mutants obtained in Example 3 and strain CHCC16165 were grown in 9.6% B-milk containing 0.01% sucrose. After acidification, the milk acidified with CHCC16165 had a lactose concentration of 14.9, a galactose concentration of 8.4 and a glucose concentration of 5.7. In comparison the milk acidified with the best performing mutant of CHCC16165 had a lactose concentration of 9.3, a galactose concentration of 10.5 and a glucose concentration of 9.9. The said best mutant of CHCC16165 was designated CHCC16731 and it was subjected to further testing in fermentation of milk when used in combination with *Lactobacillus delbrueckii* subsp. *bulgaricus* strains CHCC16159 and CHCC16160 (described in WO2013/160413).

Overnight cultures of CHCC16165 and CHCC16731 in combination with each of CHCC16159 and CHCC16160 were added to 200 ml samples of B-milk and acidified at 40°C.

The results were as listed in Table 1:

| Strain blend | Lactose mg/ml | Galactose mg/ml | Glucose mg/ml |
|---|---|---|---|
| CHCC16165 + CHCC16159 | 14.0 | 10.9 | 6.5 |
| CHCC16165 + CHCC16160 | 13.5 | 11.0 | 6.9 |
| CHCC16731 + CHCC16159 | 1.0 | 22.0 | 16.2 |
| CHCC16731 + CHCC16160 | 1.0 | 22.5 | 16.0 |
| B-milk | 48.9 | Not detectable | Not detectable |

As will appear from the results of Table 1, CHCC16731 is capable of removing almost all of the lactose from the milk. Also, CHCC16731 produces and secretes high levels of both galactose and glucose into the milk. Taking into account that sucrose is a 100 reference, and the sweetness of lactose is 16, the sweetness of galactose is 32 and the sweetness of glucose is 74.3, the fermented milk product produced with CHCC16731 has a highly increased sweetness.

### EXAMPLE 5. Production of a galactose positive mutant of Streptococcus thermophilus CHCC12339. (Example not according to the invention and presented for illustrative purposes only)

The *Streptococcus thermophilus* CHCC12339 was grown overnight in M17 containing 2% lactose at 40°C. An overnight culture was plated (100 µl) on a M17 plate containing 2% galactose and incubated at 40°C anaerobically. 22 colonies appear on the M17 containing 2% galactose plates. 16 of these are re-streaked on the same type of plates, and 8 of these are transferred to liquid M17 containing 2% galactose, all of which result in growth, and 4 of these cultures are frozen. 2 of the frozen cultures are spread on M17 plates containing 2% galactose and 40 mM 2-deoxyglucose, and for each culture 8 of the resulting colonies were selected. The 16 2-deoxyglucose mutants were transferred to liquid M17 containing 2% lactose and 2% galactose and grown overnight at 40°C, after which the mutants were grown in liquid M17 containing 2% galactose for 3 nights and the production of galactose and glucose was measured and followed through the growth. Based on the production of galactose and glucose 7 cultures were selected for milk acidification testing by adding 2 ml culture to 200 ml 9.5% B-milk containing 0.05% sucrose. 3 mutants have acidification profiles resembling that of the mother strain CHCC12339, and those 3 mutants are streaked to single colonies and incubated for 48 hours. Subsequently, the colonies are transferred to liquid M17 containing 2% galactose and the growth for the 3 mutants is tested against CH12339. The mutant having the highest growth is selected and purified once more by streaking to single colony 3 times to produce a purified galactose positive strain named CHCC18948 (galactose positive variant of CHCC12339).

### EXAMPLE 6. Production of a 2-deoxyglucose mutant of Streptococcus thermophilus CHCC18948 with enhanced excretion of glucose. (Example not according to the invention and presented for illustrative purposes only)

100 µl of the CHCC18948 culture is spread on M17 plates containing 2% galactose and 20 mM or 40 mM 2-deoxyglucose and incubated overnight at 40°C. 8 colonies from the 20 mM plates and 8 colonies from the 40 mM plates are selected and grown in liquid M17 containing 2% galactose and 20 mM 2-oxyglucose. The resulting cultures are frozen and re-streaked, and 12 of the resulting colonies are started in M17 medium and used to acidify B-milk. 1% of an overnight culture of the 2-deoxyglucose mutant is inoculated into 200 ml 9.5% B-milk containing 0.05% sucrose and acidified at 40°C. All 12 2-deoxyglucose mutants of CHCC18948 produced fermented mill products with low concentrations of lactose and high concentrations of galactose and glucose. 7 mutants were selected for retesting alone and for testing together with *Lactobacillus delbrueckii* subsp. *bulgaricus* strains CHCC16159 (described in WO2013/160413). Again 1% of an overnight culture of the 2-deoxyglucose mutant is inoculated into 200 ml 9.5% B-milk containing 0.05% sucrose and acidified at 40°C.

The results are given in Table 2:

| Strain / strain blend | Lactose mg/ml | Galactose mg/ml | Glucose mg/ml |
|---|---|---|---|
| CHCH18948-Mutant1 | Not detectable | 17.9 | 20.8 |
| CHCH18948-Mutant2 | Not detectable | 18.9 | 23.8 |
| CHCH18948-Mutant3 | 9.5 | 13.6 | 14.5 |
| CHCH18948-Mutant7 | Not detectable | 19.1 | 24.2 |
| CHCH18948-Mutant10 | 1.7 | 16.0 | 21.1 |
| CHCH18948-Mutant11 | Not detectable | 17.5 | 22.9 |
| CHCH18948-Mutant12 | 3.6 | 16.9 | 18.7 |
| CHCH18948-Mutant1 + CHCC16159 | 7.9 | 16.7 | 9.6 |
| CHCH18948-Mutant2 + CHCC16159 | 9.1 | 18.4 | 10.3 |
| CHCH18948-Mutant3 + CHCC16159 | 3.5 | 18.6 | 11.9 |
| CHCH18948-Mutant7 + CHCC16159 | 10.7 | 15.1 | 7.7 |
| CHCH18948-Mutant10 + CHCC16159 | 10.2 | 16.2 | 8.5 |
| CHCH18948-Mutant11 + CHCC16159 | 10.5 | 15.9 | 8.4 |
| CHCH18948-Mutant12 + CHCC16159 | 3.3 | 19.0 | 10.9 |
| B-milk | 47.1 | Not detectable | Not detectable |

The three best mutants are mutants 2, 3 and 12, and they are re-tested in B-milk containing 0.05% sucrose alone and in combination with *Lactobacillus delbrueckii* subsp. *bulgaricus* strains CHCC16159, CHCC16160 and CHCC16161 (described in WO2013/160413)

The results are given in Table 3:

| Strain / strain blend | Lactose mg/ml | Galactose mg/ml | Glucose mg/ml |
|---|---|---|---|
| CHCH16159 | 9.9 | 14.8 | 8.7 |
| CHCH16160 | 1.6 | 18.5 | 17.5 |
| CHCH16161 | 12.8 | 13.7 | 8.6 |
| CHCH18948-Mutant2 + CHCH16159 | 6.8 | 19.4 | 12.0 |
| CHCH18948-Mutant2 + CHCH16160 | Not detectable | 21.3 | 17.6 |
| CHCH18948-Mutant2 + CHCH16161 | Not detectable | 20.5 | 17.1 |
| CHCH18948-Mutant12 + CHCH16159 | 5.0 | 19.3 | 12.1 |
| CHCH18948-Mutant12 + CHCC16160 | 1.4 | 21.8 | 16.5 |
| CHCH18948-Mutant12 + CHCC16161 | 4.2 | 20.2 | 14.6 |
| CHCH18948-Mutant3 + CHCC16159 | 1.6 | 19.8 | 13.2 |
| CHCH18948-Mutant3 + CHCC16160 | Not detectable | 20.2 | 16.5 |
| CHCH18948-Mutant3 + CHCC16161 | 3.0 | 19.5 | 16.5 |
| CHCH18948-Mutant2 | Not detectable | 19.7 | 27.3 |
| CHCH18948-Mutant3 | Not detectable | 19.9 | 27.1 |
| CHCH18948-Mutant12 | 4.7 | 15.1 | 17.0 |
| B-milk | 42.8 | Not detectable | Not detectable |

Based on the above data CHCH18948-Mutant3 was selected as the best mutant and it was designated CHCC19216. As will appear from the above data CHCC19216 is capable of removing almost all of the lactose from the milk. Also, CHCC19216 produces and secretes high levels of both galactose and glucose into the milk. Taking into account that sucrose is a 100 reference, and the sweetness of lactose is 16, the sweetness of galactose is 32 and the sweetness of glucose is 74.3, the fermented milk product produced with CHCC19216 has a highly increased sweetness.

### EXAMPLE 7. Texturizing property of CHCC16731 and CHCC19216. (Example not according to the invention and presented for illustrative purposes only)

The texturizing property of strains CHCC16731 and CHCC19216 were tested by measurement of the shear stress by use of the following assay:
Seven days after incubation, the fermented milk was brought to 13°C and stirred gently by means of a stick fitted with a bored disc until homogeneity of the sample. The rheological properties of the sample were assessed on a rheometer (Anton Paar Physica ASC/DSR301 Rheometer (Autosampler), Anton Paar^{®} GmbH, Austria) using the following settings:
Wait time (to rebuild to somewhat original structure)
5 minutes without oscillation or rotation
Oscillation (to measure G' and G" for calculation of G^{∗})
   y = 0.3%, frequency (f) = [0.5...8] Hz
   6 measuring points over 60 s (one every 10 s)
Rotation (to measure shear stress at 300 1/s etc.)
y = [0.2707-300] 1/s and y = [275-0.2707] 1/s
21 measuring point over 210 s (one every 10 s) going up to 300 1/s
   and
21 measuring points over 210 s (one every 10 s) going down to 0.2707 1/s
For further analysis the shear stress at 300 1/s was chosen.

The strains CHCC16731 and CHCC19216 in the blends listed in Table 4 were used to acidify samples of 200 ml 9.5% B-milk using an inoculum of 0.024% until the pH reached 4.55.

The results are given in Table 4:

| Blend No. | Content of St of invention | Content of St CHCC16404 | Content of St CHCC15757 | Content of Lb CHCC-16159 | Shear stress Pa |
|---|---|---|---|---|---|
| 1 | 72.73% CHCC1673 1 | 13.64% | 4.54% | 9.09% | 41.3 |
| 2 | 72.73% CHCC1921 6 | 13.64% | 4.54% | 9.09% | 51.5 |
| 3 | 36.36% CHCC1673 1 + 33.36% CHCC1921 6 | 13.64% | 4.54% | 9.09% | 43.3 |

| | | | | | |
|---|---|---|---|---|---|
| Lb: L. delbruecki spp. Bulgaricus strain CHCC16159. St: Streptococcus thermophilus. | | | | | |

As will appear from the results, strains CHCC16731 and CHCC19216 when used as sole S. thermophilus in the culture blend, produce a fermented milk product with a shear stress of 41.3 Pa and 51.5 Pa, respectively. When used in combination strains CHCC16731 and CHCC19216 produce a fermented milk product with a shear stress of 43.3 Pa.

This level of shear stress is high as compared to conventional sweetening strains as disclosed in e.g. WO2013/160413.

### EXAMPLE 8. Flavor and growth of Lactobacillus delbrueckii subsp. bulgaricus (Lb) for culture composition of the invention culture compared to culture with lactose-positive, glucose-positive Lb

### Experimental plan

**Table 5: Composition of cultures tested**

| | CHCC16404 (St) g | CHCC16731 (St) g | CHCC15757 (St) g | CHCC18944 (Lb) g | CHCC12813 (Lb) g |
|---|---|---|---|---|---|
| 1: Culture of invention | 10.5 | 10.5 | 6 | 1.5 | 0 |
| 2: Culture with conventional Lb | 10.5 | 10.5 | 6 | 0 | 1.5 |

The culture of the invention is composed of three glucose-deficient *Streptococcus thermophilus* (St) and one lactose-deficient Lb. Culture 2 (comparative culture) is composed of three glucose-deficient St and one lactose-positive, glucose-positive (conventional) Lb.

The two cultures with the composition indicated in Table 5 were prepared and used as starter culture in a fermentation to produce yogurt at 43°C until a target pH of 4.55 was reached. The two cultures were compared with respect to flavor, growth of Lb, post-acidification, carbohydrates and rheology.

### Milk Base

The milk base contained 4.5% protein, 1.0% fat and 0.1% sucrose.

0.5% fat milk and 1.5% fat milk were blended in order to achieve a content of 1% fat in the final milk base and 0.1% sucrose was added. Protein levels were adjusted to 4.5% using skimmed milk powder (SMP). Hydration took place at 6°C for 2 hours. Thereafter, the milk was homogenized at 200/50 bar at 65°C and pasteurized at 95°C for 5 minutes.

### Yoghurt production parameters

pH during fermentation was measured online using Intap. When the yogurts reached pH 4.55 the gel was broken using a stick with a bored disc. The gel was then treated in a post-treatment unit (PTU) at 2 bars at 25°C, filled into 120 mL yogurt cups and stored at 6°C for 28 days.

### Measurement of shear stress

The method described in Example 7 was used with the exception that the samples were not stirred with a stick fitted with a bored stick, since the gel had already been broken during the PTU treatment to obtain homogeneity of the samples.

### Measurement of Colony Forming units

M17 was used as selective medium for St. MRSlac (MRS + 8% lactose) was used as selective medium for Lb. A 10X dilution series was made of the yogurts. Every dilution was plated on M17 and MRSlac agar plates, respectively and incubated anaerobically at 37°C for 2 days. Thereafter, colony forming units were counted.

### Acidification

Culture 1 took 10.8 hours to reach a pH of 4.55. Culture 2 took 12.1 hours to reach a pH of 4.55.

### Post-acidification

**Table 6: Post-acidification**

| pH | Day 0 | Day 1 | Day 7 | Day 14 | Day 21 | Day 28 |
|---|---|---|---|---|---|---|
| Culture 1 | 4.55 | 4.53 | 4.51 | 4.54 | 4.50 | 4.54 |
| Culture 2 | 4.55 | 4.51 | 4.50 | 4.51 | 4.50 | 4.54 |

### Growth of Lb

**Table 7: Culture growth (Colony Forming Units (CFU)) at the end of fermentation**

| | Lb (CFU) | St (CFU) |
|---|---|---|
| Culture 1 | 3E+07 | 9E+08 |
| Culture 2 | 9E+05 | 8E+08 |

As will appear from Table 7 the Lb cell count of the culture of the invention was much higher than the Lb count of the comparative culture with a conventional Lb, i.e. a lactose-positive, glucose-positive Lb. Thus, the combination of glucose-deficient St and lactose-deficient Lb produces a higher level of Lb at the end of the fermentation.

### Rheology

Culture 1 has a shear stress at the end of fermentation at 300 1/s of 41.1 Pa. Culture 2 has a shear stress at the end of fermentation at 300 1/s of 27.3 Pa.

### Carbohydrates

**Table 8: Level of carbohydrates at the end of fermentation**

| | Glucose (mg/g) | Galactose (mg/g) | Lactose (mg/g) |
|---|---|---|---|
| Culture 1 | 15.3 | 13.8 | 31.1 |
| Culture 2 | 15.3 | 13.6 | 13.3 |

### Flavor

Flavor was assessed by evaluation by a tasting panel. Culture 1 did not have any off-flavors.

### EXAMPLE 9. Flavor and growth of Lactobacillus delbrueckii subsp. bulgaricus (Lb) for culture composition of the invention culture compared to culture with glucose-deficient Lb

### Experimental plan

**Table 9: Composition of cultures tested**

| | CHCC16404 (St) g | CHCC16731 (St) g | CHCC15757 (St) g | CHCC18944 (Lb) g | CHCC16159 (Lb) g |
|---|---|---|---|---|---|
| 1: Culture of invention | 10.5 | 10.5 | 6 | 1.5 | 0 |
| 2: Culture with glucose-deficient Lb | 10.5 | 10.5 | 6 | 0 | 1.5 |

The culture of the invention is composed of three glucose-deficient St and one lactose-deficient Lb. Culture 2 (comparative culture) is composed of three glucose-deficient St and one lactose-positive, glucose-deficient Lb.

The two cultures with the composition indicated in Table 9 were prepared and used as starter culture in a fermentation to produce yogurt at 43°C until a target pH of 4.55 was reached. The two cultures were compared with respect to flavor (level of amino acids), volatile organic components, carbohydrates and rheology.

### Milk Base

Two milk bases were used adjusted with either Skimmed Milk Powder (SMP) or whey protein (WP) and contained 4.5% protein, 1% fat and 0.1% sucrose. 0.5% fat milk and 1.5% fat milk were blended in order to achieve a content of 1% fat in the final milk base and 0.1% sucrose was added. Protein levels were adjusted to 4.5% using SMP or WP. Hydration took place at 6°C for 2 hours. Thereafter, the milk was homogenized at 200/50 bar at 65°C and pasteurized at 95°C for 5 minutes.

### Yoghurt production parameters

pH during fermentation was measured online in the baby bottles using CINAC. When the yogurts reached pH 4.55 the gel was broken and homogenized using a stick with a bored disc. Thereafter, the yoghurt was stored in baby bottles.

### Measurement of shear stress

The method described in Example 7 was used.

### Rheology

Culture 1 in SMP milk base has a shear stress at the end of fermentation at 300 1/s of 46.2 Pa.
Culture 1 in WP milk base has a shear stress at the end of fermentation at 300 1/s of 39.6 Pa.
Culture 2 in SMP milk base has a shear stress at the end of fermentation at 300 1/s of 42.0 Pa.
Culture 2 in WP milk base has a shear stress at the end of fermentation at 300 1/s of 38.4 Pa.

As will appear from the results culture 1 has a higher shear stress as compared to culture 2.

### Post-acidification

**Table 10: Post- acidification**

| pH | Day 0 | Day 7 | Day 14 | Day 21 | Day 28 |
|---|---|---|---|---|---|
| Culture 1 in SMP milk base | 4.55 | 4.51 | 4.56 | 4.39 | 4.49 |
| Culture 1 in WP milk base | 4.55 | 4.52 | 4.55 | 4.39 | 4.48 |
| Culture 2 in SMP milk base | 4.55 | 4.45 | 4.50 | 4.32 | 4.43 |
| Culture 2 in WP milk base | 4.55 | 4.42 | 4.47 | 4.30 | 4.40 |

### Growth of Lb

**Table 11: Culture growth (Colony Forming Units (CFU)) at the end of fermentation**

| | LB (CFU) | ST (CFU) |
|---|---|---|
| Culture 1 in SMP milk base | 1E+07 | 2E+08 |
| Culture 1 in WP milk base | 2E+07 | 5E+07 |
| Culture 2 in SMP milk base | 2E+08 | 9E+07 |
| Culture 2 in WP milk base | 2E+08 | 1E+08 |

As will appear from Table 11 the Lb cell count of the culture of the invention was lower than the Lb count of the comparative culture with a lactose-positive, glucose-deficient Lb.

### Carbohydrates

**Table 12: Level of carbohydrates at the end of fermentation**

| | Glucose (mg/g) | Galactose (mg/g) | Lactose (mg/g) |
|---|---|---|---|
| Culture 1 in SMP milk base | 14.4 | 13.2 | 15.4 |
| Culture 1 in WP milk base | 12.0 | 10.2 | 9.2 |
| Culture 2 in SMP milk base | 16.4 | 16.3 | 11.7 |
| Culture 2 in WP milk base | 14.6 | 14.1 | 6.1 |

### Amino acids

**Table 13: Level of amino acids**

| | Glutamic acid (ppm) | Valine (ppm) | Average of Total Free Amino Acids (mM) |
|---|---|---|---|
| Culture 1 in SMP milk base | 77 | 9 | 5.5 |
| Culture 1 in WP milk base | 62 | 23 | 4.4 |
| Culture 2 in SMP milk base | 137 | 22 | 7.1 |
| Culture 2 in WP milk base | 85 | 45 | 5.4 |

Samples containing the glucose-negative Lb (Cultures 2) have a higher amount of TFFA, which points towards higher proteolytic activity, which in turn can lead to unpleasant flavors.

Furthermore, yogurts containing the glucose-negative Lb contain a higher amount of glutamic acid which is known for giving off-flavors described as 'brothy'.

Valine, a bitter amino acid, is as well present in higher amounts in yogurts containing the glucose-negative Lb.

### Volatile organic compounds (VOC)

A VOC profile can give indications for the flavor profile of a sample. 51 different volatile organic compounds have been measured. For Culture 1 in WP milk base, the amount of volatile organic compound was lower for 44 out of 51 compounds (86%) as compared to Culture 2 in WP milk base. For Culture 1 in WP milk base, the amount of volatile organic compound was lower for 34 out of 51 compounds (67%) as compared to Culture 2 in WP milk base. In conclusion the cultures of the invention had a significantly lower content of volatile organic compounds (viewed as a group of compounds) as compared to the comparative cultures.

### EXAMPLE 10. Growth and post-acidification of Lactobacillus delbrueckii subsp. bulgaricus (Lb) for culture composition of the invention

### Experimental plan

**Table 14: Composition of culture tested**

| | CHCC16404 (St) % | CHCC16731 (St) % | CHCC15757 (St) % | CHCC18944 (Lb) % |
|---|---|---|---|---|
| 1: Culture of invention | 36.8 | 36.8 | 21.1 | 5.2 |

The culture with the composition indicated in Table 14 was prepared and used as starter culture in a fermentation to produce yogurt at 43°C until a target pH of 4.55 was reached. The culture was inoculated at a level of 0.01 %, 0.02% and 0.03%. The yogurt produced was stored at 5°C, 13°C and 25°C. The culture was tested with respect to growth of St and Lb and post-acidification.

### Milk bases

**Table 15: Composition of milk bases 1 and 2**

| | Amount | Protein | Carbohydrate | Fat |
|---|---|---|---|---|
| Skim milk | 884.2 g | | | |
| 1.5 % milk | 1767.1 g | | | |
| Promilk 802FB | 10.9 g | | | |
| SMP | 73.9 g | | | |
| Sucrose | 2.8 g | | | |
| Water | 261.0 g | | | |
| Milk Base | 3000 g | 4.57% | 5.61% | 0.92% |

**Table 16: Composition of milk base 3**

| | Amount | Protein | Carbohydrate | Fat |
|---|---|---|---|---|
| Skim milk | 884.2 g | | | |
| 1.5 % milk | 1767.1 g | | | |
| Promilk 802FB | 10.9 g | | | |
| SMP | 73.9 g | | | |
| Sucrose | 6.0 g | | | |
| Water | 257.8 g | | | |
| Milk Base | 3000 g | 4.57% | 5.71% | 0.92% |

### Post-acidification

**Table 17: Post-acidification**

| Milk base | 1 | 1 | 1 | 3 | 3 | 3 |
|---|---|---|---|---|---|---|
| Inoculation | 0.01% | 0.02% | 0.03% | 0.01% | 0.02% | 0.03% |
| 5°C Day 1 | 4.54 | 4.54 | 4.53 | 4.53 | 4.53 | 4.52 |
| 5°C Day 7 | 4.50 | 4.50 | 4.49 | 4.51 | 4.51 | 4.51 |
| 5°C Day 21 | 4.48 | 4.48 | 4.49 | 4.50 | 4.50 | 4.50 |
| 5°C Day 42 | 4.43 | 4.42 | 4.45 | 4.43 | 4.45 | 4.42 |
| 13°C Day 1 | 4.50 | 4.49 | 4.50 | 4.51 | 4.50 | 4.50 |
| 13°C Day 7 | 4.46 | 4.46 | 4.45 | 4.50 | 4.49 | 4.50 |
| 13°C Day 21 | 4.38 | 4.38 | 4.38 | 4.42 | 4.45 | 4.42 |
| 25°C Day 1 | 4.43 | 4.43 | 4.43 | 4.45 | 4.46 | 4.45 |
| 25°C Day 7 | 4.40 | 4.40 | 4.40 | 4.43 | 4.44 | 4.43 |

As will appear from Table 17, the level of post-acidification is very low for all inoculation levels and storage temperatures tested, including the higher storage temperatures of 13°C and 25°C, where the level of growth of Lb and hence the post-acidification is high for most conventional starter cultures.

### Growth of Lb and St

**Table 18: Growth of Lb and St**

| Milk base | 1 | 1 | 1 | 2 | 2 | 2 |
|---|---|---|---|---|---|---|
| Inoculation | 0.01% | 0.02% | 0.03% | 0.01% | 0.02% | 0.03% |
| Lb CFU/g | 5.8E05 | 6.5E05 | 7.2E05 | 3.7E05 | 4.7E05 | 8.8E05 |
| St CFU/g | 4.2E07 | 6.5E07 | 7.4E07 | 3.8E07 | 5.6E07 | 7.0E07 |

As will appear from Table 18, for all levels of inoculation, the Lb cell count was at a high level at the end of the fermentation.

### EXAMPLE 11. Growth, post-acidification and carbohydrate analysis of Lactobacillus delbrueckii subsp. bulgaricus (Lb) for culture compositions of the invention compared to conventional starter cultures.

### Experimental plan

**Table 19: Composition of cultures tested**

| | Sample | CHCC16404 (St) % | CHCC16731 (St) % | CHCC15757 (St) % | CHCC18944 (Lb) % | Sucrose addition after fermentation |
|---|---|---|---|---|---|---|
| 1 | YoFlex Premium 1.0 | | | | | 6.0% |
| 2 | Yoflex YF-L901 | | | | | 6.0% |
| 3 | Culture of invention | 36.8 | 36.8 | 21.1 | 5.2 | 5.0% |
| 4 | Culture of invention | 36.8 | 36.8 | 21.1 | 5.2 | 4.5% |
| 5 | Culture of invention | 36.8 | 36.8 | 21.1 | 5.2 | 5.5% |
| 6 | Culture of invention | 36.8 | 36.8 | 21.1 | 5.2 | 4.8% |

The cultures with the composition indicated in Table 19 were prepared and used as starter culture in a fermentation to produce yogurt at 43°C until a target pH of 4.55 was reached. The culture was inoculated at a level of 0.02%. The yogurt produced was stored at 5°C and 13°C. The cultures were tested with respect to growth of St and Lb, post-acidification and carbohydrate analysis.

YoFlex Premium 1.0 and Yoflex YF-L901 are commercial, conventional cultures comprising lactose-positive and glucose-positive St and Lb.

### Milk base

**Table 20: Composition of milk base 1**

| | Amount | Protein | Carbohydrate | Fat |
|---|---|---|---|---|
| 1.5 % milk | 3000 g | | | |
| SMP | 85 g | | | |
| Milk Base | 3085 g | 4.20% | 6.06% | 1.49% |

### Post-acidification

**Table 21: Post-acidification**

| Sample | 1 | 2 | 3 | 4 | 5 | 6 |
|---|---|---|---|---|---|---|
| 6°C Day 1 | 4.49 | 4.48 | 4.54 | 4.54 | 4.53 | 4.54 |
| 6°C Day 7 | 4.45 | 4.43 | 4.52 | 4.52 | 4.52 | 4.53 |
| 6°C Day 21 | 4.44 | 4.37 | 4.47 | 4.47 | 4.46 | 4.46 |

As will appear from Table 21 the cultures of the invention have a low level of post-acidification, which is at the same level as the conventional starter cultures.

### Growth of Lb and St

**Table 22: Growth of Lb and St**

| Sample | 1 | 2 | 3 | 4 | 5 | 6 |
|---|---|---|---|---|---|---|
| Lb CFU/g | 6.1E04 | 1.7E05 | 3.1E06 | 2.8E06 | 3.1E06 | 2.8E06 |
| St CFU/g | 3.0E08 | 4.1E08 | 3.2E08 | 3.1E08 | 3.0E08 | 3.1E08 |

As will appear from Table 22, the Lb cell count for the cultures of the invention was at a higher level at the end of the fermentation than the conventional cultures.

### Carbohydrate analysis

**Table 23: Carbohydrate analysis**

| | Sample | Galactose mg/g | Glucose mg/g | Lactose mg/g | Sucrose mg/g |
|---|---|---|---|---|---|
| 1 | YoFlex Premium 1.0 | 6.35 | <LOD | 34.35 | 46.55 |
| 2 | Yoflex YF-L901 | 8.05 | 1.00 | 33.05 | 46.60 |
| 3 | Culture of invention | 8.95 | 8.70 | 19.90 | 41.55 |
| 4 | Culture of invention | 8.90 | 8.75 | 19.70 | 37.50 |
| 5 | Culture of invention | 8.60 | 8.40 | 19.15 | 43.10 |
| 6 | Culture of invention | 8.80 | 8.65 | 19.40 | 39.65 |

### LOD: Limit of Detection

All determinations of carbohydrates were made in duplicate, and Table 23 lists the average value of the two determinations. The determinations were carried out after 7 days of storage of the yogurt.

As will appear from Table 23, the cultures of the invention produced glucose at a high level, and after 7 days of storage the glucose was present at a level of approx. 8 mg/g.

### DEPOSI TS AND EXPERT SOLUTI ONS

The applicant requests that a sample of the deposited micro-organisms stated below may only be made available to an expert, until the date on which the patent is granted.

The strain *Streptococcus thermophilus* CHCC11342 that was deposited at Deutsche Sammlung von Mikroorganismen und Zellkulturen (DSMZ) GmbH, Inhoffenstr. 7B, D-38124 Braunschweig, Germany, on 8 September 2009 under the accession No. DSM 22932.

The strain *Streptococcus thermophilus* CHCC12339 has been deposited at Deutsche Sammlung von Mikroorganismen und Zellkulturen (DSMZ) GmbH, Inhoffenstr. 7B, D-38124 Braunschweig, Germany, on 14 October 2010 under the accession No. DSM 24090.

The strain *Streptococcus thermophilus* CHCC11976 has been deposited at Deutsche Sammlung von Mikroorganismen und Zellkulturen (DSMZ) GmbH, Inhoffenstr. 7B, D-38124 Braunschweig, Germany, on 8 September 2009 under the accession No. DSM 22934.

The strain *Streptococcus thermophilus* CHCC16731 has been deposited at Deutsche Sammlung von Mikroorganismen und Zellkulturen (DSMZ) GmbH, Inhoffenstr. 7B, D-38124 Braunschweig, Germany, on 4 June 2014 under the accession No. DSM 28889.

The strain *Streptococcus thermophilus* CHCC19216 has been deposited at Deutsche Sammlung von Mikroorganismen und Zellkulturen (DSMZ) GmbH, Inhoffenstr. 7B, D-38124 Braunschweig, Germany, on 8 December 2015 under the accession No. DSM 32227.

The strain *Streptococcus thermophilus* CHCC15757 strain that was deposited at Deutsche Sammlung von Mikroorganismen und Zellkulturen (DSMZ) GmbH, Inhoffenstr. 7B, D-38124 Braunschweig, Germany, on 3.4.2012 under the accession No. DSM 25850.

The strain *Streptococcus thermophilus* CHCC15887 strain that was deposited at Deutsche Sammlung von Mikroorganismen und Zellkulturen (DSMZ) GmbH, Inhoffenstr. 7B, D-38124 Braunschweig, Germany, on 3.4.2012 under the accession No. DSM 25851.

The strain Streptococcus thermophilus CHCC16404 strain that was deposited at Deutsche Sammlung von Mikroorganismen und Zellkulturen (DSMZ) GmbH, Inhoffenstr. 7B, D-38124 Braunschweig, Germany, on 12.12.2012 under the accession No. DSM 26722.

The strain Lactobacillus delbrueckii subsp. bulgaricus CHCC18944 deposited with DSMZ-Deutsche Sammlung von Mikroorganismen und Zellkulturen GmbH, Inhof-fenstr. 7B, D-38124 Braunschweig, on 2014-06-12 under the accession no. DSM 28910.

The strain Lactobacillus delbrueckii subsp. bulgaricus CHCC10019 deposited with DSMZ-Deutsche Sammlung von Mikroorganismen und Zellkulturen GmbH, Inhoffenstr. 7B, D-38124 Braunschweig on 2007-04-03 under the accession No. DSM 19252.

The strain Lactobacillus delbrueckii subsp. bulgaricus CHCC16159 deposited with DSMZ-Deutsche Sammlung von Mikroorganismen und Zellkulturen GmbH, Inhoffenstr. 7B, D-38124 Braunschweig on 2012-09-06 under the accession No. DSM 26420.

The strain Lactobacillus delbrueckii subsp. bulgaricus CHCC16160 deposited with DSMZ-Deutsche Sammlung von Mikroorganismen und Zellkulturen GmbH, Inhoffenstr. 7B, D-38124 Braunschweig on 2012-09-06 under the accession No. DSM 26421.

The strain Lactobacillus delbrueckii subsp. bulgaricus CHCC12813 deposited with DSMZ-Deutsche Sammlung von Mikroorganismen und Zellkulturen GmbH, Inhoffenstr. 7B, D-38124 Braunschweig on 2010-09-29 under the accession No. DSM 24074.

The deposits were made according to the Budapest treaty on the international recognition of the deposit of microorganisms for the purposes of patent procedure.

### REFERENCES

WO2011/026863
WO2011/092300
WO2013/160413
Pool et al. (2006) Metabolic Engineering 8(5); 456-464
Thompson et al. (1985) J. Bacteriol. 162(1); 217-223
Chervaux et al. (2000). Appl and Environ Microbiol, 66, 5306-5311
Cochu et al. (2003). Appl and Environ Microbiol, 69(9), 5423-5432
Høier et al. (2010) in The Technology of Cheesemaking, 2nd Ed. Blackwell Publishing, Oxford; 166-192.

## Claims

1. Composition for producing a fermented milk product comprising:
(i) at least one *Streptococcus thermophilus* (St) strain, wherein the St strain is galactose-fermenting, wherein the strain carries a mutation in the DNA sequence of the *glcK* gene encoding a glucokinase protein, wherein the mutation inactivates the glucokinase protein or has a negative effect on expression of the gene, wherein the galactose-fermenting capacity is determined as disclosed in the description and wherein the mutation reduces the activity of the glucokinase protein with at least 50% and wherein the glucokinase activity is determined as indicated in the description. and
(ii) at least one *Lactobacillus delbrueckii* subsp. *bulgaricus* (Lb) strain, wherein the Lb strain is lactose-deficient and capable of metabolizing glucose, wherein lactose-deficiency is determined via the capacity of the Lb strain to form white colonies on a medium containing lactose and 5-bromo-4-chloro-3-indolyl-beta-D-galacto-pyranoside (X-Gal).

2. The composition according to claim 1, wherein the St strain is 2-deoxyglucose resistant.

3. The composition according to claim 1 or 2, wherein the St strain carries a mutation that reduces the transport of glucose into the cell.

4. The composition according to any of the preceding claims, wherein the St strain increases the amount of glucose in 9.5% B-milk to at least 5 mg/ml when inoculated into the 9.5% B-milk at a concentration of 10⁶-10⁷ CFU/ml and grown at 40°C for 20 hours.

5. The composition according to any of the preceding claims, wherein the St strain increases the amount of glucose in 9.5% B-milk with 0.05% sucrose to at least 5 mg/ml when inoculated into the 9.5% B-milk with 0.05% sucrose at a concentration of 10⁶-10⁷ CFU/ml and grown at 40°C for 20 hours.

6. The composition according to any of the preceding claims, wherein the Lb strain is selected from the group consisting of the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28910, and a Z functionally equivalent mutant strain derived from DSM 28910, wherein less than 1% of the nucleotides in the bacterial genome have been replaced with another nucleotide, or deleted, compared to the mother strain.

7. A composition according to any of claims 1-6, wherein the St strain is selected from the group consisting of the *Streptococcus thermophilus* CHCC19216 strain that was deposited at Deutsche Sammlung von Mikroorganismen und Zellkulturen under accession No. DSM 32227, the strain *Streptococcus thermophilus* CHCC16731 has been deposited at Deutsche Sammlung von Mikroorganismen und Zellkulturen (DSMZ) GmbH, Inhoffenstr. 7B, D-38124 Braunschweig, Germany, on 4 June 2014 under the accession No. DSM 28889, the *Streptococcus thermophilus* CHCC15757 strain that was deposited at Deutsche Sammlung von Mikroorganismen und Zellkulturen under accession No. DSM 25850, a *Streptococcus thermophilus* CHCC15887 strain that was deposited at Deutsche Sammlung von Mikroorganismen und Zellkulturen under accession No. DSM 25851, the *Streptococcus thermophilus* CHCC16404 strain that was deposited at Deutsche Sammlung von Mikroorganismen und Zellkulturen under accession No. DSM 26722, and a mutant strain derived therefrom, wherein the mutant strain Z is a functionally equivalent mutant of the deposited strains and wherein less than 1% of the nucleotides in the bacterial genome have been replaced with another nucleotide, or deleted, compared to the mother strain.

8. A method for producing a fermented milk product comprising inoculating and fermenting a milk substrate with the composition according to any one of claims 1 to 7.

9. A fermented milk product comprising the composition according to any one of claims 1 to 7.

10. Use of the composition according to any one of claims 1 to Z 7 for the preparation of a fermented milk product.

## Patentansprüche

1. Zusammensetzung zur Herstellung eines fermentierten Milchprodukts, umfassend:
(i) mindestens einen *Streptococcus thermophilus*(St)-Stamm, wobei der St-Stamm Galactose-fermentierend ist, wobei der Stamm eine Mutation in der DNA-Sequenz des glcK-Gens trägt, das für ein Glucokinaseprotein codiert, wobei die Mutation das Glucokinaseprotein inaktiviert oder einen negativen Effekt auf die Expression des Gens hat, wobei die Galactose-Fermentationskapazität wie in der Beschreibung angegeben bestimmt wird und wobei die Mutation die Aktivität des Glucokinaseproteins um mindestens 50 % reduziert und wobei die Glucokinaseaktivität wie in der Beschreibung angegeben bestimmt wird, und
(ii) mindestens einen *Lactobacillus delbrueckii* subsp. *bulgaricus(Lb)-Stamm,* wobei der Lb-Stamm Lactose-defizient und in der Lage ist, Glucose zu metabolisieren, wobei Lactose-Mangel über die Fähigkeit des Lb-Stamms bestimmt wird, weiße Kolonien auf einem Medium zu bilden, das Lactose und 5-Brom-4-chlor-3-indolyl-beta-D-galactopyranosid (X-Gal) enthält.

2. Zusammensetzung nach Anspruch 1, wobei der St-Stamm 2-Desoxyglucose-resistent ist.

3. Zusammensetzung nach Anspruch 1 oder 2, wobei der St-Stamm eine Mutation trägt, die den Transport von Glucose in die Zelle reduziert.

4. Zusammensetzung nach einem der vorhergehenden Ansprüche, wobei der St-Stamm die Glucosemenge in 9,5 % B-Milch auf mindestens 5 mg/ml erhöht, wenn 9,5 % B-Milch bei einer Konzentration von 10⁶-10⁷ KBE/ml damit beimpft wird und er 20 Stunden bei 40 °C gezüchtet wird.

5. Zusammensetzung nach einem der vorhergehenden Ansprüche, wobei der St-Stamm die Glucosemenge in 9,5 % B-Milch mit 0,05 % Saccharose auf mindestens 5 mg/ml erhöht, wenn die 9,5 % B-Milch mit 0,05 % Saccharose bei einer Konzentration von 10⁶-10⁷ KBE/ml damit beimpft wird und er 20 Stunden bei 40 °C gezüchtet wird.

6. Zusammensetzung nach einem der vorhergehenden Ansprüche, wobei der Lb-Stamm ausgewählt ist aus der Gruppe bestehend aus dem Stamm, der bei der DSMZ - Deutsche Sammlung von Mikroorganismen und Zellkulturen GmbH, Inhoffenstr. 7B, D-38124 Braunschweig am 12.06.2014 unter der Zugangsnr. DSM 28910 hinterlegt wurde, und einem funktionell äquivalenten mutierten Stamm, abgeleitet von DSM 28910, wobei weniger als 1 % der Nukleotide im Bakteriengenom im Vergleich zum Mutterstamm durch ein anderes Nukleotid ersetzt oder deletiert wurden.

7. Zusammensetzung nach einem der Ansprüche 1-6, wobei der St-Stamm ausgewählt ist aus der Gruppe bestehend aus dem *Streptococcus thermophilus*-CHCC19216-Stamm, der bei der Deutschen Sammlung von Mikroorganismen und Zellkulturen unter der Zugangsnr. DSM 32227 hinterlegt wurde, dem Stamm *Streptococcus thermophilus* CHCC16731, der bei der Deutschen Sammlung von Mikroorganismen und Zellkulturen (DSMZ) GmbH, Inhoffenstr. 7B, D-38124 Braunschweig, Deutschland, am 4. Juni 2014 unter der Zugangsnr. DSM 28889 hinterlegt wurde, dem *Streptococcus thermophilus* CHCC15757-Stamm, der bei der Deutschen Sammlung von Mikroorganismen und Zellkulturen unter der Zugangsnr. DSM 25850 hinterlegt wurde, einem *Streptococcus thermophilus* CHCC15887-Stamm, der bei der Deutschen Sammlung von Mikroorganismen und Zellkulturen unter der Zugangsnr. DSM 25851 hinterlegt wurde, dem *Streptococcus thermophilus* CHCC16404-Stamm, der bei der Deutschen Sammlung von Mikroorganismen und Zellkulturen unter der Zugangsnr. DSM 26722 hinterleget wurde, und einem davon abgeleiteten mutierten Stamm, wobei der mutierte Stamm eine funktionell äquivalente Mutante der hinterlegten Stämme ist und wobei weniger als 1 % der Nukleotide im Bakteriengenom im Vergleich zum Mutterstamm durch ein anderes Nukleotid ersetzt oder deletiert wurden.

8. Verfahren zur Herstellung eines fermentierten Milchprodukts, umfassend ein Beimpfen und Fermentieren eines Milchsubstrats mit der Zusammensetzung nach einem der Ansprüche 1 bis 7.

9. Fermentiertes Milchprodukt, umfassend die Zusammensetzung nach einem der Ansprüche 1 bis 7.

10. Verwendung der Zusammensetzung nach einem der Ansprüche 1 bis 7 zur Herstellung eines fermentierten Milchprodukts.

## Revendications

1. Composition pour produire un produit à base de lait fermenté comprenant :
(i) au moins une souche *Streptococcus thermophilus* (St), dans laquelle la souche St fermente le galactose, dans laquelle la souche porte une mutation dans la séquence d'ADN du gène *glcK* codant pour une protéine glucokinase, dans laquelle la mutation inactive la protéine glucokinase ou a un effet négatif sur l'expression du gène,
dans laquelle la capacité de fermentation du galactose est déterminée comme divulgué dans la description et
dans laquelle la mutation réduit l'activité de la protéine glucokinase d'au moins 50 % et dans laquelle l'activité de la glucokinase est déterminée comme indiqué dans la description, et
(ii) au moins une souche *Lactobacillus delbrueckii* subsp. *bulgaricus* (Lb), dans laquelle la souche Lb est déficiente en lactose et est capable de métaboliser le glucose, dans laquelle la déficience en lactose est déterminée via la capacité de la souche Lb à former des colonies blanches sur un milieu contenant du lactose et du 5-bromo-4-chloro-3-indolyl-bêta-D-galacto-pyranoside (X-Gal).

2. Composition selon la revendication 1, dans laquelle la souche St est résistante au 2-désoxyglucose.

3. Composition selon la revendication 1 ou 2, dans laquelle la souche St porte une mutation qui réduit le transport de glucose dans la cellule.

4. Composition selon l'une quelconque des revendications précédentes, dans laquelle la souche St augmente la quantité de glucose dans du lait B à 9,5 % jusqu'à au moins 5 mg/mL lorsqu'elle est inoculée dans le lait B à 9,5 % à une concentration de 10⁶-10⁷ CFU/mL et mise à croître à 40 °C pendant 20 heures.

5. Composition selon l'une quelconque des revendications précédentes, dans laquelle la souche St augmente la quantité de glucose dans du lait B à 9,5 % avec du saccharose à 0,05 % jusqu'à au moins 5 mg/mL lorsqu'elle est inoculée dans le lait B à 9,5 % avec du saccharose à 0,05 % à une concentration de 10⁶-10⁷ CFU/mL et mise à croître à 40 °C pendant 20 heures.

6. Composition selon l'une quelconque des revendications précédentes, dans laquelle la souche Lb est choisie dans le groupe consistant en la souche déposée auprès de DSMZ-Deutsche Sammlung von Mikroorganismen und Zellkulturen GmbH, Inhoffenstr. 7B, D-38124 Braunschweig, le 12-06-2014 sous le numéro d'accès DSM 28910, et une souche mutante fonctionnellement équivalente dérivée de DSM 28910, dans laquelle moins de 1 % des nucléotides dans le génome bactérien ont été remplacés par un autre nucléotide, ou supprimés, en comparaison à la souche mère.

7. Composition selon l'une quelconque des revendications 1 à 6, dans laquelle la souche St est choisie dans le groupe consistant en la souche *Streptococcus thermophilus* CHCC19216 qui a été déposée auprès de Deutsche Sammlung von Mikroorganismen und Zellkulturen sous le numéro d'accès DSM 32227, la souche *Streptococcus thermophilus* CHCC16731 a été déposée auprès de Deutsche Sammlung von Mikroorganismen und Zellkulturen (DSMZ) GmbH, Inhoffenstr. 7B, D-38124 Braunschweig, Allemagne, le 4 juin 2014 sous le numéro d'accès DSM 28889, la souche *Streptococcus thermophilus* CHCC15757 qui a été déposée auprès de Deutsche Sammlung von Mikroorganismen und Zellkulturen sous le numéro d'accès DSM 25850, une souche *Streptococcus thermophilus* CHCC15887 qui a été déposée auprès de Deutsche Sammlung von Mikroorganismen und Zellkulturen sous le numéro d'accès DSM 25851, la souche *Streptococcus thermophilus* CHCC16404 qui a été déposée auprès de Deutsche Sammlung von Mikroorganismen und Zellkulturen sous le numéro d'accès DSM 26722, et une souche mutante dérivée de celles-ci, dans laquelle la souche mutante est un mutant fonctionnellement équivalent des souches déposées et dans laquelle moins de 1 % des nucléotides dans le génome bactérien ont été remplacés par un autre nucléotide, ou supprimés, en comparaison à la souche mère.

8. Procédé pour produire un produit à base de lait fermenté comprenant l'inoculation et la fermentation d'un substrat de lait avec la composition selon l'une quelconque des revendications 1 à 7.

9. Produit à base de lait fermenté comprenant la composition selon l'une quelconque des revendications 1 à 7.

10. Utilisation de la composition selon l'une quelconque des revendications 1 à 7 pour la préparation d'un produit à base de lait fermenté.
